# EUROPÄISCHE PATENTSCHRIFT

(11) **EP 0 665 289 B1**
(45) Veröffentlichungstag und Bekanntmachung des Hinweises auf die Patenterteilung: **23.05.2007**
(21) Anmeldenummer: 95100764.0
(22) Anmeldetag: 20.01.1995
(51) Int. Cl.: C12N 9/88, C07K 7/08, C07K 16/40, G01N 33/68, C12N 15/07, A61K 38/04, A61K 38/51

(54) **Autoimmunreaktion hervorrufende GAD65 Peptide**
Peptides from GAD65, provoking autoimmune reactions
Peptides dérivés de GAD65, provoquant des réactions autoimmunes

(30) Priorität: 20.01.1994 DE 4401629; 04.02.1994 DE 4403522; 24.05.1994 DE 4418091
(43) Veröffentlichungstag der Anmeldung: 02.08.1995
(73) Patentinhaber: Roche Diagnostics GmbH, 68305 Mannheim (DE)
(72) Erfinder: Endl, Josef, Dr., D-82362 Weilheim (DE); Stahl, Pter, Dr., D-82347 Bernried (DE); Albert, Winfried, Dr., D-82390 Eberfing (DE); Jung, Günther-Gerhard, Prof. Dr., D-72076 Tübingen (DE); Schendel, Dolores J., Prof. Dr., D-80469 München (DE); Meinl, Edgar, Dr., D-82152 Krailling (DE); Dornmair, Klaus, Dr., D-82275 Emmering (DE)
(74) Vertreter: Weiss, Wolfgang

(56) Entgegenhaltungen:
- EP-A- 0 519 469
- WO-A-92/18150
- WO-A-92/20811
- WO-A-94/04557
- WO-A-94/12529
- WO-A-95/07992
- J. IMMUNOL., Bd. 152, Nr. 2, 15.Januar 1994 Seiten 930-934, LI, L. ET AL. 'Identification of auto- antibody epitopes of glutamic acid decarboxylase in stiff-man syndrome patients'
- J. CLIN. INVEST., Bd. 89, 1992 Seiten 1161-1165, HARRISON, L.C. ET AL. 'Islet reactive T cells are a marker of preclinical insulin dependent diabetes'
- DIABETES, Bd. 41, 1992 Seiten 782-787, CHRISTIE, M.R. ET AL. 'Antibodies to GAD ...'

### SEQUENZPROTOKOLL

<110> Roche Diagnostics GmbH
<120> Antigen-spezifische, aktivierte T-Lymphozyten, Nachweis und Anwendung
<130> 10177PEP_DR
<140> 95 100 764.0
   <141> 1995-01-20
<150> DE P 44 01 629.8
   <151> 1994-01-20
<150> DE P 44 03 522.5
   <151> 1994-02-04
<150> DE P 44 18 091.8
   <151> 1994-05-24
<160> 23
<170> Patent In Ver. 2.1
<210> 1
   <211> 25
   <212> PRT
   <213> Künstliche Sequenz
<220>
   <223> Beschreibung der künstlichen Sequenz: Autoimmunreaktion hervorrufendes GAD 65 Peptid
<400> 1
   Gly Met Ala Ala Leu Pro Arg Leu Ile Ala Phe Thr Ser Glu His Ser
     1               5                  10                  15
   His Phe Ser Leu Lys Lys Gly Ala Ala
                20                  25
<210> 2
   <211> 20
   <212> PRT
   <213> Künstliche Sequenz
<220>
   <223> Beschreibung der künstlichen Sequenz: Autoimmunreaktion hervorrufendes GAD 65 Peptid
<400> 2
   Glu Arg Gly Lys Met Ile Pro Ser Asp Leu Glu Arg Arg Ile Leu Glu
    1               5                  10                  15
   Ala Lys Gln Lys
               20
<210> 3
   <211> 14
   <212> PRT
   <213> Künstliche Sequenz
<220>
   <223> Beschreibung der künstlichen Sequenz: Autoimmunreaktion hervorrufendes GAD 65 Peptid
<400> 3
   Ile Leu Ile Lys Cys Asp Glu Arg Gly Lys Met Ile Pro Ser
    1               5                  10
<210> 4
   <211> 14
   <212> PRT
   <213> Künstliche Sequenz
<220>
   <223> Beschreibung der künstlichen Sequenz: Autoimmunreaktion hervorrufendes GAD 65 Peptid
<400> 4
   Leu Gly Ile Gly Thr Asp Ser Val Ile Leu Ile Lys Cys Asp
    1               5                  10
<210> 5
   <211> 14
   <212> PRT
   <213> Künstliche Sequenz
<220>
   <223> Beschreibung der künstlichen Sequenz: Autoimmunreaktion hervorrufendes GAD 65 Peptid
<400> 5
   Leu Ala Phe Leu Gln Asp Val Met Asn Ile Leu Leu Gln Tyr
   1 5 10
<210> 6
   <211> 14
   <212> PRT
   <213> Künstliche Sequenz
<220>
   <223> Beschreibung der künstlichen Sequenz: Autoimmunreaktion hervorrufendes GAD 65 Peptid
<400> 6
   Tyr Asp Leu Ser Tyr Asp Thr Gly Asp Lys Ala Leu Gln Cys
   1 5 10
<210> 7
   <211> 14
   <212> PRT
   <213> Künstliche Sequenz
<220>
   <223> Beschreibung der künstlichen Sequenz: Autoimmunreaktion hervorrufendes GAD 65 Peptid
<400> 7
   Val Ser Tyr Gln Pro Leu Gly Asp Lys Val Asn Phe Phe Arg
   1 5 10
<210> 8
   <211> 14
   <212> PRT
   <213> Künstliche Sequenz
<220>
   <223> Beschreibung der künstlichen Sequenz: Autoimmunreaktion hervorrufendes GAD 65 Peptid
<400> 8
   Leu Ala Ala Asp Trp Leu Thr Ser Thr Ala Asn Thr Asn Met
   1 5 10
<210> 9
   <211> 14
   <212> PRT
   <213> Künstliche Sequenz
<220>
   <223> Beschreibung der künstlichen Sequenz: Autoimmunreaktion hervorrufendes GAD 65 Peptid
<400> 9
   Leu Leu Tyr Gly Asp Ala Glu Lys Pro Ala Glu Ser Gly Gly
   1 5 10
<210> 10
   <211> 14
   <212> PRT
   <213> Künstliche Sequenz
<220>
   <223> Beschreibung der künstlichen Sequenz: Autoimmunreaktion hervorrufendes GAD 65 Peptid
<400> 10
   Val Asn Tyr Ala Phe Leu His Ala Thr Asp Leu Leu Pro Ala
   1 5 10
<210> 11
   <211> 14
   <212> PRT
   <213> Künstliche Sequenz
<220>
   <223> Beschreibung der künstlichen Sequenz: Autoimmunreaktion hervorrufendes GAD 65 Peptid
<400> 11
   Leu Leu Gln Tyr Val Val Lys Ser Phe Asp Arg Ser Thr Lys
   1 5 10
<210> 12
   <211> 14
   <212> PRT
   <213> Künstliche Sequenz
<220>
   <223> Beschreibung der künstlichen Sequenz: Autoimmunreaktion hervorrufendes GAD 65 Peptid
<400> 12
   Phe Thr Tyr Glu Ile Ala Pro Val Phe Val Leu Leu Glu Tyr
   1 5 10
<210> 13
   <211> 14
   <212> PRT
   <213> Künstliche Sequenz
<220>
   <223> Beschreibung der künstlichen Sequenz: Autoimmunreaktion hervorrufendes GAD 65 Peptid
<400> 13
   Leu Glu Tyr Val Thr Leu Lys Lys Met Arg Glu Ile Ile Gly
    1               5                   10
<210> 14
   <211> 14
   <212> PRT
   <213> Künstliche Sequenz
<220>
   <223> Beschreibung der künstlichen Sequenz: Autoimmunreaktion hervorrufendes GAD 65 Peptid
<400> 14
   Asn Met Tyr Ala Met Met Ile Ala Arg Phe Lys Met Phe Pro
    1               5                   10
<210> 15
   <211> 14
   <212> PRT
   <213> Künstliche Sequenz
<220>
   <223> Beschreibung der künstlichen Sequenz: Autoimmunreaktion hervorrufendes GAD 65 Peptid
<400> 15
   Lys Ile Trp Met His Val Asp Ala Ala Trp Gly Gly Gly Leu
    1               5                   10
<210> 16
   <211> 14
   <212> PRT
   <213> Künstliche Sequenz
<220>
   <223> Beschreibung der künstlichen Sequenz: Autoimmunreaktion hervorrufendes GAD 65 Peptid
<400> 16
   Trp Gly Gly Gly Leu Leu Met Ser Arg Lys His Lys Trp Lys
    1               5                   10
<210> 17
   <211> 14
   <212> PRT
   <213> Künstliche Sequenz
<220>
   <223> Beschreibung der künstlichen Sequenz: Autoimmunreaktion hervorrufendes GAD 65 Peptid
<400> 17
   Glu Gly Tyr Glu Met Val Phe Asp Gly Lys Pro Gln His Thr
   1               5                   10
<210> 18
   <211> 14
   <212> PRT
   <213> Künstliche Sequenz
<220>
   <223> Beschreibung der künstlichen Sequenz: Autoimmunreaktion hervorrufendes GAD 65 Peptid
<400> 18
   Arg Tyr Phe Asn Gln Leu Ser Thr Gly Leu Asp Met Val Gly
   1               5                   10
<210> 19
   <211> 14
   <212> PRT
   <213> Künstliche Sequenz
<220>
   <223> Beschreibung der künstlichen Sequenz: Autoimmunreaktion hervorrufendes GAD 65 Peptid
<400> 19
   Trp Leu Thr Ser Thr Ala Asn Thr Asn Met Phe Thr Tyr Glu
   1               5                   10
<210> 20
   <211> 14
   <212> PRT
   <213> Künstliche Sequenz '
<220>
   <223> Beschreibung der künstlichen Sequenz: Autoimmunreaktion hervorrufendes GAD 65 Peptid
<400> 20
   Thr Ala Asn Thr Asn Met Phe Thr Tyr Glu Ile Ala Pro Val
   1               5                   10
<210> 21
   <211> 14
   <212> PRT
   <213> Künstliche Sequenz
<220>
   <223> Beschreibung der künstlichen Sequenz: Autoimmunreaktion hervorrufendes GAD 65 Peptid
<400> 21
   Leu Val Ser Ala Thr Ala Gly Thr Thr Val Tyr Gly Ala Phe
    1               5                  10
<210> 22
   <211> 14
   <212> PRT
   <213> Künstliche Sequenz
<220>
   <223> Beschreibung der künstlichen Sequenz: Autoimmunreaktion hervorrufendes GAD 65 Peptid
<400> 22
   Tyr Ile Pro Pro Ser Leu Arg Thr Leu Glu Asp Asn Glu Glu
    1               5                  10
<210> 23
   <211> 14
   <212> PRT
   <213> Künstliche Sequenz
<220>
   <223> Beschreibung der künstlichen Sequenz: Autoimmunreaktion hervorrufendes GAD 65 Peptid
<400> 23
   Val Ile Ser Asn Pro Ala Ala Thr His Gln Asp Ile Asp Phe
    1               5                  10

## Beschreibung

Die vorliegende Erfindung betrifft Komplexe von Peptiden mit Molekülen des Major Histocompatibility Komplex (MHC), wobei diese Peptide eine Autoimmunreaktion hervorrufen, mit den Komplexen aus Peptiden und MHC-Molekülen reagierende T-Zellsubpopulationen sowie diagnostische und therapeutische Anwendungen dieser Verbindungen.

Die Aufklärung der molekularen Zusammenhänge bei der Entstehung von Autoimmunerkrankungen, wie etwa der rheumatoiden Arthritis und des juvenilen Diabetes (IDDM), ist innerhalb der letzten Jahre schnell fortgeschritten und läßt mittlerweile konkrete Anwendungen für die frühe Diagnose und eine kausale Therapie dieser Erkrankungen erkennen.

Heute gilt als gesichert, daß bei der Entstehung dieser Erkrankungen neben einer genetischen Disposition auch Umweltfaktoren eine Rolle spielen. Auf der Ebene der genetischen Risikofaktoren sind z.B. bei dem IDDM nur einige wenige Allele der MHC-Klasse II-Antigene eng mit dieser Erkrankung assoziiert. Somit besteht die Möglichkeit, über eine Analyse dieser Allele eine Risikogruppe für IDDM zu definieren (vgl. z.B. Thomson et al., Am. J. Hum. Genet. 43 (1988), 799-816 oder Todd et al., Nature 329 (1987), 599-604).

Bei den an der Entstehung von IDDM beteiligten Umweltfaktoren handelt es sich wahrscheinlich um exogene, als Immunogen wirksame Peptidsequenzen. In diesem Zusammenhang werden u.a. virale Antigene, die partielle Homologien zu körpereigenen Strukturen aufweisen, diskutiert. Unter besonderen Umständen, insbesondere in der postnatalen Phase, können durch die Nahrung aufgenommene Antigene, wie z.B. Rinderserumalbumin, eine Immunantwort induzieren, welche aufgrund von Homologien zu körpereigenen Strukturen einen autoaggressiven Prozeß in Gang setzen können.

Typisch für den Krankheitsverlauf bei IDDM ist die progressive Zerstörung der Pankreas-β-Zellen durch cytotoxische Lymphozyten. Dieser Prozeß setzt schon lange vor einer erkennbaren Störung des Glucosestoffwechsels ein. Bei einer erkennbaren Manifestation des Diabetes sind bereits über 90 % der β-Zellen zerstört. Es wäre deshalb außerordentlich wichtig, diese autoaggressiven T-Zellen frühzeitig bei Risikopersonen zu erfassen, um die betroffenen Individuen einer kausalen Therapie zuführen zu können.

Es gilt heute als gesichert, daß die Zerstörung von körpereigenem Gewebe bei Autoimmunerkrankungen anfänglich sehr langsam verläuft. Im Anfangsstadium dieses Prozesses erkennen die autoaggressiven T-Zellen wahrscheinlich nur ein oder wenige Autoantigene. Arbeiten von Kaufman et al. (Nature 368 (1993), 69-72) und Tisch et al. (Nature 368 (1993), 72-78) an einem Tiermodell (NOD-Maus) des Typ I-Diabetes haben ergeben, daß beim spontan auftretenden Diabetes dieses Mausstammes die initiale, über T-Zellen vermittelte Auto-Immunreaktion gegen die Glutaminsäure-Decarboxylase gerichtet ist. Dabei werden in der NOD-Maus anfänglich nur 1 bis 2 Epitope am C-Terminus der Glutaminsäure-Decarboxylase (GAD) erkannt. Zu diesem Zeitpunkt lassen sich - wie oben ausgeführt - noch keine Veränderungen im Glucose-Metabolismus feststellen, während hingegen eine Perinsulitis bereits nachweisbar ist. Erst im weiteren Krankheitsverlauf weitet sich das Spektrum der von den autoaggressiven T-Zellen erkannten Peptide der GAD aus. Nach einer Manifestation des Diabetes sind auch präaktivierte T-Zellen gegen andere Inselzell-Antigene nachweisbar, z.B. Peripherin, Heat Shock Protein HSP 65 und Carboxypeptidase H.

Es gibt Hinweise, daß auch beim Menschen die Immunantwort gegen GAD ursächlich mit dem Entstehen des Typ I-Diabetes verknüpft ist. So lassen sich beispielsweise in über 80 % der Prädiabetiker Autoantikörper gegen GAD nachweisen, wobei die ätiologische Rolle dieser Autoantikörper allerdings gering eingeschätzt wird. Man nimmt vielmehr an, daß beim Typ I-Diabetes eine progressive Zerstörung der Pankreas-β-Zellen durch T-Lymphozyten vorliegt. Diese gegen GAD gerichteten T-Lymphozyten konnten bereits von mehreren Forschergruppen nachgewiesen werden (Harrison et al., J. Clin. Invest. 89 (1992), 1161; Honeyman et al., J. Exp. Med. 177 (1993), 535). Die von diesen Gruppen gefundenen Autoantikörper reagierten mit einem aus den Aminosäuren 208 bis 404 bestehenden Peptidfragment des GAD 67 kd Moleküls.

Honeyman et al. (J. Exp. Med. 177: 535-540, 1993) beschreiben die Klonierung und Expression von einem humanen GAD 67-Autoantigen (Aminosäuren 208-404) als GST-Fusionsprotein.

In EP-A-0 519 469 werden autoimmun reagierende Polypeptide aus dem humanen GAD 65 kd Molekül offenbart. Diese Polypeptide haben die Aminosequenz:
X-P-E-V-K-(T oder E)-K-Z,
wobei X eine fakultative, aus 1 bis 10 Aminosäuren ausgewählte Sequenz ist und Z eine fakultative, aus 1 bis 8 Aminosäuren ausgewählte Sequenz ist.

Eine der vorliegenden Erfindung zugrundeliegende Aufgabe bestand darin, neue autoreaktive Peptide bereitzustellen, die mit T-Zellen aus Typ I-Diabetikern, insbesondere mit T-Zellen aus frisch entdeckten Typ I-Diabetikern reagieren und somit frühe Auto-Epitope definieren.

Diese Aufgabe wird gelöst durch Peptide, Peptid-Derivate oder analog-bindende Moleküle, die zum Nachweis, zur Isolierung, zur Vermehrung, zur Anergisierung oder/und zur Elimination autoreaktiver T-Zellen geeignet sind. Ein Gegenstand der Erfindung ist somit ein Komplex, der mindestens ein Peptid oder Peptid-Derivat gebunden an ein MHC-Molekül oder ein peptidbindendes Derivat eines MHC-Moleküls umfasst, wobei das Peptid oder Peptid-Derivat eine maximale Länge von 100 Aminosäuren aufweist und umfasst:
(a) eine der in SEQ ID NO. 1 bis SEQ ID NO. 23 gezeigten Aminosäuresequenzen,
(b) Teilbereiche der in SEQ ID NO. 1 bis SEQ ID NO. 23 gezeigten Aminosäuresequenzen mit einer Länge von mindestens 6 Aminosäuren, die eine im Wesentlichen äquivalente Spezifität oder/und Affinität der Bindung an humane MHC-Moleküle, wie die Aminosäuresequenzen aus (a) zeigen, oder/und
(c) von den in SEQ ID NO. 1 bis SEQ ID NO. 23 gezeigten Aminosäuresequenzen abgeleitete Aminosäuresequenzen, die eine im Wesentlichen äquivalente Spezifität oder/und Affinität der Bindung an humane MHC-Moleküle, wie die Aminosäuresequenzen aus (a) zeigen, wobei die von den in SEQ ID NO. 1 bis SEQ ID NO. 23 gezeigten Aminosäuresequenzen abgeleiteten Aminosäuresequenzen eine Sequenzhomologie von mindestens 30 % zu den Ausgangsaminosäuresequenzen aufweisen,
wobei
die Aminosäuresequenz (I)
G-M-A-A-L-P-R-L-I-A-F-T-S-E-H-S-H-F-S-L-K-K-G-A-A
SEQ ID NO:1 und
die Aminosäuresequenz (II)
E-R-G-K-M-I-P-S-D-L-E-R-R-I-L-E-A-K-Q-K.
SEQ ID NO:2 ist.

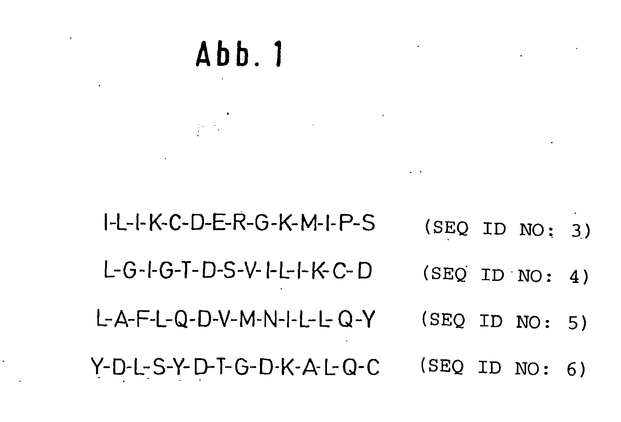

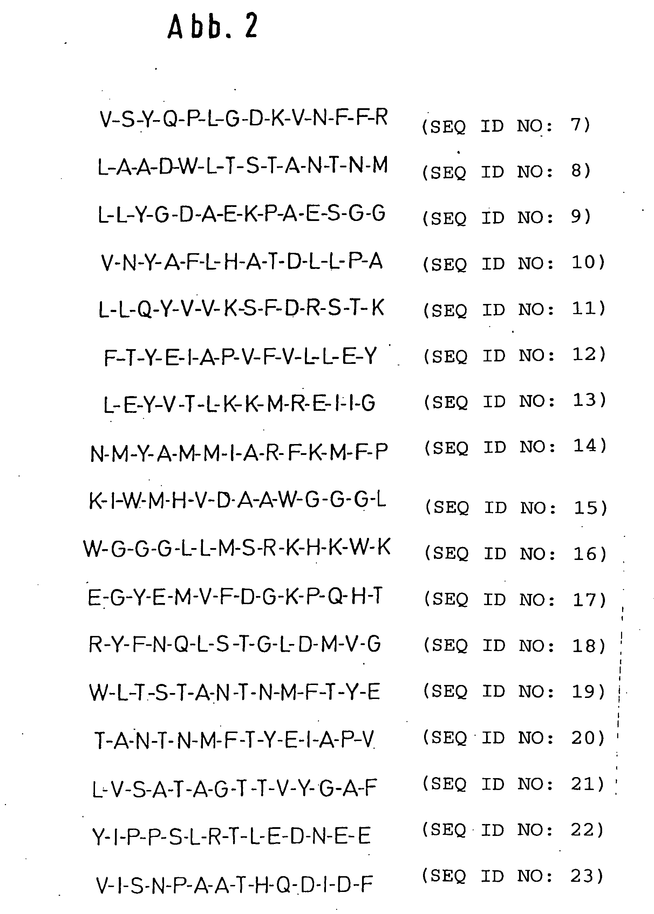

Die Sequenzen 4 bis 23 sind in den Abb. 1 und 2 dargestellt.

Vorzugsweise umfaßt der erfindungsgemäße Komplex ein Peptid oder Peptid-derivat, umfassend
(a) die in SEQ ID NO. 1 gezeigte Aminosäuresequenz,
(b) die in SEQ ID NO. 2 gezeigte Aminosäuresequenz,
(c) Teilbereiche der in SEQ ID NO. 1 oder/und SEQ ID NO. 2 gezeigten Aminosäuresequenzen, die eine im Wesentlichen ' äquivalente Spezifität oder/und Affinität der Bindung an humane MHC-Moleküle, wie die Aminosäuresequenzen aus (a) oder/und (b) zeigen, oder/und
(d) von den in SEQ ID NO. 1 oder/und SEQ ID NO. 2 gezeigten Aminosäuresequenzen abgeleitete Aminosäuresequenzen, die eine im Wesentlichen äquivalente Spezifität oder/und Affinität der Bindung an humane MHC-Moleküle, wie die Aminosäuresequenzen aus (a) oder/und (b) zeigen, wobei die von den in SEQ ID NO. 1 oder/und SEQ ID NO. 2 gezeigten Aminosäuresequenzen abgeleiteten Aminosäuresequenzen eine Sequenzhomologie von mindestens 30 % zu den Ausgangsaminosäuresequenzen aufweisen.

Besonders bevorzugt umfaßt im erfindungsgemäßben Komplex ein Peptid oder Peptid-Derivat den Teilbereich
L-P-R-L-I-A-F-T-S-E-H-S-H-F
der Aminosäuresequenz (I) oder eine davon abgeleitete Sequenz, bei der die N-terminale Sequenz L-P und die C-terminale Sequenz H-F konserviert sind.

Die Aminosäuresequenz (I) entspricht den Aminosäureresten 266 - 290 der humanen GAD 65 und die Aminosäuresequenz (II) der Aminosäuresequenz 306 - 325 der humanen GAD 65. Die in den Abbildungen 1 und 2 dargestellten Aminosäuresequenzen sind ebenfalls Teilsequenzen der humanen GAD 65.

Überraschenderweise wurde festgestellt, daß Peptide, welche den Aminosäuresequenzen 266 bis 285 und 306 bis 325 der humanen GAD 65 entsprechen, eine spezifische Reaktion mit T-Zellsubpopulationen zeigten, die aus frisch entdeckten Typ I-Diabetikern isoliert wurden. Somit handelt es sich bei den erfindungsgemäßen Peptiden um frühe Autoepitope, mit deren Verwendung eine sehr frühe Diagnose des Typ I-Diabetes ermöglicht wird. Weiterhin können die erfindungsgemäßen Peptide auch therapeutisch angewendet werden, indem die mit den Peptiden reaktive T-Zellpopulation ausgeschaltet wird.

Bevorzugte Beispiele für T-Zellsubpopulationen, mit denen die erfindungsgemäßen Peptide der Aminosäuresequenzen (I) und (II) reagieren, sind die T-Zellinien 6/7 und 6/10 oder T-Zellen mit einer äquivalenten Bindungsspezifität. Die T-Zellinien 6/7 und 6/10 wurden am 10. Mai 1994 bei der Deutschen Sammlung von Mikroorganismen und Zellkulturen GmbH (DSM), Mascheroder Weg 1b, 38124 Braunschweig, BRD unter den Nummern DSM ACC2172 (6/7) bzw. DSM ACC2173 (6/10) nach den Vorschriften des Budapester Vertrages hinterlegt.

Die Aminosäuresequenzen (I) und (II) sind Teilbereiche aus der 65 kD Isoform der humanen Glutaminsäure-Decarboxylase (GAD), deren vollständige Aminosäuresequenz von Bu et al. (Proc. Natl. Acad. Sci. USA 89 (1992), 2115 ff.) beschrieben wurde. Die Aminosäuresequenzen (I) und (II) wurden durch Anlegen von T-Zellinien aus dem peripheren Blut von Typ I-Diabetikern und anschließende in vitro Stimulation mit GAD aus Schweinehirn und Testen dieser T-Zellinien in einem Proliferationsassay mit synthetischen Peptidsequenzen gefunden, die aus der humanen GAD-Sequenz abgeleitet wurden.

Die erfindungsgemäßen Peptide können durch bekannte Syntheseverfahren mittels chemischer Methoden erzeugt werden oder durch Klonierung und Expression einer für diese Peptide codierenden DNA-Sequenz in einer geeigneten Wirtszelle, insbesondere E.coli, auf gentechnische Weise hergestellt werden.

Weiterhin umfaßt die vorliegende Erfindung auch Komplexe umfassend Peptide mit Teilbereichten der spezifisch angegebenen Aminosäuresequenzen (I) oder (II) oder der in den Abbildungen 1 und 2 dargestellten Aminosäuresequenzen, die eine Länge von mindestens 6 Aminosäuren, vorzugsweise von mindestens 8 Aminosäuren, besonders bevorzugt von mindestens 10 Aminosäuren und am meisten bevorzugt von mindestens 15 Aminosäuren aufweisen. Die minimale Länge eines erfindungsgemäßen Peptids wird durch seine Fähigkeit bestimmt, ein MHC-Molekül zu erkennen, mit ihm spezifisch zu binden und mit dem entsprechenden T-zellrezeptor zu reagieren.

Die maximale Länge der aus der GAD stammenden und MHC-bindenden Abschnitte in einem erfindungsgemäßen Peptid beträgt 100 Aminosäuren, bevorzugt 50 Aminosäuren und am meisten bevorzugt 25 Aminosäuren.

Neben Komplexen umfassend Peptide mit den Aminosäuresequenzen (I) und (II) oder Teilbereichen davon betrifft die Erfindung auch noch Komplexe umfassend Peptide mit Aminosäuresequenzen, die im wesentlichen äquivalente Spezifität oder/und Affinität der Bindung an MHC-Moleküle wie die zuvor genannten Sequenzen zeigen und die vorzugsweise durch Substitution, Deletion oder Insertion einzelner Aminosäurereste oder kurzer Abschnitte von Aminosäureresten aus den Aminosäuresequenzen (I) oder (II) abgeleitet sind oder analog bindende verfremdete Substanzen.

Insbesondere betrifft die vorliegende Erfindung auch Komplexe umfassend Peptidvarianten, die in ihrer Sequenz mit den oben genannten Aminosäuresequenzen nicht völlig übereinstimmen, sondern nur gleiche oder nahe verwandte "Ankerpositionen" aufweisen. Die Bezeichnung "Ankerposition" bedeutet in diesem Zusammenhang einen für die Bindung an ein MHC-Molekül, insbesondere an ein MHC-Molekül der Klassen DR3, DR4 oder DQ, wesentlichen Aminosäurerest. Die Ankerposition für das DRB10401-Bindungsmotiv sind z.B. bei Hammer et al., Cell 74 (1993), 197-203, angegeben. Derartige Ankerpositionen sind in erfindungsgemäßen Peptiden konserviert oder gegebenenfalls durch Aminosäurereste mit chemisch sehr nahe verwandten Seitenketten ersetzt (z.B. Alanin durch Valin, Leucin durch Isoleucin und umgekehrt). Die Bestimmung der Ankerpositionen in den erfindungsgemäßen Peptiden kann auf einfache Weise durch Tests von Varianten der oben angegebenen spezifischen Peptide auf ihre Bindungsfähigkeit an MHC-Moleküle erfolgen. Erfindungsgemäße Peptide sind dadurch gekennzeichnet, daß sie eine im wesentlichen äquivalente Spezifität oder/und Affinität der Bindung an MHC-Moleküle wie die zuvor genannten Peptide zeigen. Vorzugsweise besitzen die aus Peptiden mit den Aminosäuresequenzen (I) oder (II) oder den in den Abbildungen 1 und 2 dargestellten Aminosäuresequenzen abgeleiteten Peptide eine Sequenzhomologie von mindestens 30 %, besonders bevorzugt von mindestens 50 % und am meisten bevorzugt mindestens 60 % mit den Ausgangspeptiden oder Teilsequenzen davon.

Beispiele für Varianten der spezifisch angegebenen Peptide sind die entsprechenden homolögen Peptidabschnitte aus der humanen GAD 67, deren vollständige Aminosäuresequenz ebenfalls von Bu et al., supra, beschrieben wurde.

Der Begriff "im wesentlichen äquivalente Spezifität oder/und Affinität der Bindung an MHC-Moleküle" umfaßt auch eine gegenüber den Aminosäuresequenzen (I), (II) oder den in den Abbildungen 1 und 2 dargestellten Aminosäuresequenzen verbesserte Bindungsspezifität oder/und -affinität, die insbesondere bei verkürzten Peptiden gefunden wird, die eine Länge von vorzugsweise 8 bis 15 Aminosäuren besitzen.

Weiterhin umfaßt die vorliegende Erfindung auch Komplexe umfassend Peptid-Derivate. Dieser Begriff umfaßt Peptide, in denen eine oder mehrere Aminosäuren durch eine chemische Reaktion derivatisiert worden sind. Beispiele von erfindungsgemäßen Peptid-Derivaten sind insbesondere solche Moleküle, in denen das Backbone oder/und reaktive Aminosäureseitengruppen, z.B. freie Aminogruppen, freie Carboxylgruppen oder/und freie Hydroxylgruppen, derivatisiert worden sind. Spezifische Beispiele für Derivate von Aminogruppen sind Sulfonsäure- oder Carbonsäureamide, Thiourethanderivate und Ammoniumsalze, z.B. Hydrochloride. Beispiele für Carboxylgruppenderivate sind Salze, Ester und Amide. Beispiele für Hydroxylgruppenderivate sind O-Acyl- oder O-Alkylderivate. Weiterhin umfaßt der Begriff Peptid-Derivat gemäß vorliegender Erfindung auch solche Peptide, in denen eine oder mehrere Aminosäuren durch natürlich vorkommende oder nicht natürlich vorkommende Aminosäurehomologe der 20 "Standard"-Aminosäuren ersetzt werden. Beispiele für solche Homologe sind 4-Hydroxyprolin, 5-Hydroxylysin, 3-Methylhistidin, Homoserin, Ornithin, β-Alanin und 4-Aminobuttersäure.

Insbesondere sind Komplexe umfassend solche Peptide bevorzugt, welche eine im wesentlichen äquivalente Spezifität oder/und Affinität der

Bindung an MHC-Moleküle wie Peptide mit den Aminosäuresequenzen (I) oder (II) aufweisen, die aber im Gegensatz zu diesen Peptiden keine Aktivierung von T-Zellen, sondern die Erzeugung eines anergen Zustands in T-Zellen hervorrufen.

Von der vorliegenden Erfindung werden auch Komplexe umfassend Polypeptide erfaßt, in denen der MHC-bindende Peptidabschnitt Bestandteil einer größeren Pölypeptideinheit ist, wobei die Verbindung von MHC-bindendem Peptid und dem Rest der Polypeptideinheit vorzugsweise eine Sollbruchstelle aufweist, z.B. eine Proteasespaltstelle.

Ein weiterer Gegenstand der vorliegenden Erfindung ist ein Komplex umfassend ein Peptid oder Peptid-Derivat, das eine signalerzeugende Substanz bzw. eine Markierungsgruppe, z.B. eine Fluoreszenzmarkierungsgruppe (z.B: Rhodamin, Phycoerythrin), Digoxin, Biotin, eine radioaktive Gruppe oder eine Toxingruppe (z.B. Ricin, Choleratoxin etc.) trägt. Durch Kopplung des erfindungsgemäßen Peptids; mit Markierungsgruppen kann das Peptid als diagnostisches Mittel für in vivo oder in vitro (z.B. Imaging) Anwendungen oder als therapeutisches Mittel eingesetzt werden. Weiterhin kann das erfindungsgemäße Peptid auch beispielsweise in cyclisierter Form oder in oligomerer Form vorliegen, wobei die für die Bindung an das MHC-Molekül wichtigen Sequenzen durch Spacerregionen voneinander getrennt sind.

Die Erfindung betrifft auch Komplexe umfassend peptidmimetische Substanzen, die eine im wesentlichen äquivalente Spezifität oder/und Affinität der Bindung an MHC-Moleküle wie die zuvor genannten Peptide oder Peptid-Derivate zeigen. Peptidmimetische Substanzen oder Peptidmimetika sind Verbindungen, die Peptide in ihrer Wechselwirkung mit den MHC-Molekülen ersetzen können und gegenüber den nativen Peptiden eine erhöhte metabolische Stabilität, bessere Bioverfügbarkeit und größere Wirkungsdauer aufweisen können. Methoden zur Herstellung von Peptidmimetika sind beschrieben bei Giannis und Kolter, Angew. Chem. 105 (1993), 1303-1326, Lee et al., Bull. Chem. Soc. Jpn. 66 (1993), 2006-2010 und Dorsch et al., Kontakte (Darmstadt) (1993) (2), 48-56. Bezüglich der Herstellung erfindungsgemäßer peptidmimetischer Substanzen wird auf die Offenbarung dieser Literaturstellen verwiesen.

Ein Gegenstand der vorliegenden Erfindung ist ein Komplex, der mindestens ein erfindungsgemäßes Peptid, Peptid-Derivat oder Peptidmimetikum und mindestens ein MHC-Molekül oder ein peptidbindendes Derivat eines MHC-Moleküls umfaßt. In diesem Komplex ist ein Peptid, Peptid-Derivat oder Peptidmimetikum mit einer Bindungskonstante von vorzugsweise mindestens 10⁻⁷ 1/mol, besonders bevorzugt im Bereich von 10⁻⁸ - 10⁻⁹ 1/mol, an ein MHC-Molekül oder ein peptidbindendes Derivat eines MHC-Moleküls gebunden. Alternativ kann das Peptid, Peptid-Derivat oder Peptidmimetikum auch kovalent an das MHC-Molekül gekoppelt sein, z.B. über einen Photolinker oder als kovalente genetische Peptid-MHC-Fusion. Ein derartiges Peptid-MHC-Fusionsprotein enthält vorzugsweise eine HLA-DR beta-Kette und ein damit genetisch fusioniertes autoreaktives Peptid. Besonders bevorzugt enthält der Komplex ein MHC-Klasse 11-Molekül oder ein peptidbindendes Derivat davon.

Das MHC-Klasse II-Molekül ist vorzugsweise vom Typ DR, beispielsweise vom Typ DR1, DR2, DR3 oder DR4. Besonders bevorzugt ist das MHC-Klasse II-Molekül vom Subtyp DR B1 0101, DR B1 0301, DR B1 0401, DR B1 0402, DR B1 0404 oder DR B1 1601. Am meisten bevorzugt ist das MHC-Klasse II-Molekül vom Subtyp DR Bl 0101 oder DR B1 0401. Die T-Zellinie 6/7 (DSM ACC2172) proliferiert mit dem autoreaktiven Peptid der Aminosäuresequenz (I) in Anwesenheit der DR B1-Allele 0401 und 0101 oder/und 1601. Mit dem autoreaktiven Peptid der Aminosäuresequenz (II) wird eine Proliferation in Gegenwart des DR B1-Allels 0401 gefunden. Die T-Zellinie 6/10 (DSM ACC2173) proliferiert mit den autoreaktiven Peptiden der Aminosäuresequenzen (I) und (II) in Gegenwart des DR B1-Allels 0401.

Die Nukleotidsequenzen der für ein MHC-Klasse 11-Molekül der obigen Subtypen kodierenden Gene sind veröffentlicht in Corell et al. (Mol. Immunol. 28 (1991), 533-543). Auf den Inhalt dieser Publikation wird hiermit Bezug genommen.

Der Begriff "peptibindendes Derivat eines MHC-Moleküls" umfaßt Fragmente von MHC-Molekülen, die durch proteolytische Spaltung nativer MHC-Moleküle oder durch rekombinante DNA-Techniken hergestellt sind und ihre peptidbindenden Eigenschaften im wesentlichen beibehalten haben. Weiterhin sind unter diesem Begriff Fusionsproteine zu verstehen, die neben einem für die Peptidbindung verantwortlichen MHC-Anteil noch weitere Polypeptid-Komponenten enthalten.

Die erfindungsgemäßen Peptid-MHC-Komplexe werden vorzugsweise durch Assoziierung peptidfreier MHC-Moleküle oder MHC-Molekülderivate mit den erfindungsgemäßen Peptiden, Peptid-Derivaten oder Peptidmimetika hergestellt. Die Herstellung von peptidfreien MHC-Molekülen kann beispielsweise durch Entfaltung von nativen MHC-Molekülen, um gebundene Peptide zu dissoziieren, und Rückfaltung der leeren MHC-Moleküle erfolgen (siehe Dornmair und McConnell, Proc. Natl. Acad. Sci. USA 87 (1990), 4134-4138 und WO91/14701).

Andererseits können peptidfreie MHC-Moleküle auch durch rekombinante Herstellung von MHC-Molekülen oder Derivaten davon gewonnen werden. Beispiele hierfür sind die Expression von MHC-Klasse II-Molekülen in Fibroblasten (Germain und Malissen, Ann. Rev. Immunol. 4 (1990), 281-315) sowie die Expression von löslichen MHC-Klasse II-Molekülderivaten ohne Membrananker in CHO-Zellen (Wettstein et al., J. Exp. Med. 174 (1991), 219-228, Buelow et al., Eur. J. Immunol. 23 (1990), 69-76) und mittels des Baculovirus-Expressionssystems in Insektenzellen (Stern und Wiley, Cell 68 (1992), 465-477; Scheirle et al., J. Immunol. 149 (1992), 1994-1999). Auch MHC-Klasse I-Moleküle wurden in CHO-Zellen (Fahnestock et al., Science 258 (1992), 1658-1662) in Insektenzellen (Jackson et al., Proc. Natl. Acad. Sci. USA 89 (1992), 12117-12120; Matsamura et al., J. Biol. Chem. 267 (1992), 23589-23595) sowie in Fibroblasten (Mage et al., Proc. Natl. Acad. Sei. USA 89 (1992), 10658-10661) exprimiert.

Weiterhin ist auch die Expression von peptidfreien MHC-Molekülen in E.coli bekannt (Parker et al., Mol. Immunol. 29 (1992), 371-378; Zhang et al., Proc. Natl. Acad. Sci. USA 89 (1992), 8403-8407; Garboczi et al., Proc. Natl. Acad. Sci. USA 89 (1992), 3429-3433; Altman et al., Proc. Natl. Acad. Sci. USA 90 (1993), 10330-10334). Auf die in diesen Veröffentlichungen beschriebenen Techniken zur rekombinanten Expression von MHC-Molekülen oder MHC-Molekülderivaten wird für die vorliegende Erfindung Bezug genommen.

Vorzugsweise ist der MHC-Bestandteil des erfindungsgemäßen Komplexes ein rekombinantes MHC-Molekül oder ein peptidbindendes Derivat davon und besonders bevorzugt ein lösliches MHC-Molekülderivat, bei dem der Membrananker teilweise oder vollständig deletiert ist.

Zur Identifizierung von MHC-Molekülen, welche das erfindungsgemäße autoreaktive Peptid präsentieren, werden die Antigen präsentierenden Zellen eines Spenders mit dem erfindungsgemäßen Peptid in markierter Form inkubiert, wobei vorzugsweise zuerst gebundene Peptide durch Denaturierung nativer MHC-Moleküle dissoziiert werden. Anschließend können die markierten MHC-Peptid-Komplexe mit Subtyp-spezifischen Antikörpern, die gegen Framework-spezifische Determinanten der MHC-Moleküle gerichtet sind, immunpräzipitiert und aufgrund des Vorhandenseins der markierten Peptide identifiziert werden.

Alternativ können als Antigen präsentierende Zellen auch EBV (Epstein-Barr-Virus) transformierte B-Zellen des Spenders verwendet werden.

Die Herstellung der erfindungsgemäßen Komplexe aus einem rekombinanten MHC-Molekülderivat kann beispielsweise so erfolgen, daß DNA-Fragmente für die löslichen Teile der α- und β-Ketten eines MHC-Moleküls, z.B. eines MHC-DR3-, DR4- oder DQ-Moleküls durch PCR isoliert werden, wobei als Template cDNA aus einer EBV transformierten B-Zellinie des Spenders benutzt wird, welche das entsprechende MHC-Molekül exprimiert. Bei diesem Schritt wird vorzugsweise am C-Terminus der α- und der β-Kette durch entsprechende Auswahl des PCR-Primers eine Reinigungshilfe, z.B. ein Oligohistidinsegment (z.B. ein Hexa-Histidin-Segment), eingeführt. Die PCR-Produkte können anschließend in E. coli subkloniert und als Inclusion-Bodies exprimiert werden. Die Inclusion-Bodies können nach bekannten Verfahren (vgl. Literaturstellen zur Expression von MHC-Molekülen in E.coli, supra) solubilisiert und die MHC-Proteine mittels MetallChelat-Affinitätschromatographie gereinigt werden. Anschließend werden die α- und β-Untereinheiten in Anwesenheit des Peptids renaturiert.

Der erfindungsgemäße Peptid-MHC-Komplex kann auch eine wie oben beschriebene Markierungsgruppe tragen, wobei die Markierungsgruppe sowohl am Peptidbestandteil als auch am MHC-Bestandteil des Komplexes durch bekannte Methoden gebunden sein kann.

Ein weiterer Gegenstand der vorliegenden Erfindung ist ein oligomerisierter Peptid-MHC-Komplex, der mindestens 2 MHC-Moleküle oder MHC-Molekülderivate enthält, die über kovalente oder nicht-kovalente Wechselwirkungen assoziiert sind. Ein derartiger oligomerisierter Peptid-MHC-Molekül-Komplex hat gegenüber bekannten (bezüglich des MHC-Moleküls) monomeren Komplexen den Vorteil einer höheren Affinität und somit einer verbesserten diagnostischen oder/und therapeutischen Wirksamkeit.

In einer Ausführungsform der vorliegenden Erfindung kann ein derartiger oligomerisierter Komplex durch kovalente Quervernetzung von monomeren Peptid/MHC-Molekül-Komplexen über chemische Kopplungsreagenzien, z.B. N-Succinimidyl-3(2-pyridylthio)propionat, 3-Maleimidobenzoyl-N-hydroxy-succinimidester, Maleimidohexanoyl-N-hydroxy-succinimidester, Bis(maleimidomethyl)ether, Disuccinimidylsuberat, Glutardialdehyd etc. nach bekannten Verfahren hergestellt werden. Gegebenenfalls können auch einzelne Aminosäuren der Peptidkomponente oder der MHC-Komponente so verändert sein, daß spezielle Kopplungsreagenzien an dieser Stelle bevorzugt angreifen. So lassen sich durch Einführung von zusätzlichen Cystein- oder Lysin-Resten auf rekombinantem Wege bei der Proteinkomponente bzw. durch chemische Synthese bei der Peptidkomponente Kopplungen über SH-Linker bzw. über Aminogruppen erzielen.

In einer weiteren Ausführungsform der vorliegenden Erfindung kann der oligomerisierte Peptid-MHC-Komplex so hergestellt werden, daß die an das MHC-Molekül bindende Peptidkomponente als Oligomer eingesetzt wird, d.h. als ein Peptidmolekül, das mindestens 2 MHC-bindende Bereiche enthält, wobei die für die Bindung an das MHC-Molekül wichtigen Sequenzen durch Spacerregionen voneinander getrennt sind. Diese Spacerregionen bestehen üblicherweise aus 10 - 15 Aminosäuren. Man verwendet kleine, hydrophile Aminosäuren, z.B. Glycin, Alanin, Serin, Prolin bzw. Kombinationen davon. Bei einer Renaturierung von peptidfreien MHC-Molekülen in Anwesenheit dieser Peptidoligomere entsteht der erfindungsgemäße oligomerisierte Komplex, der durch die oligomerisierte Peptidkomponente über nicht-kovalente Wechselwirkungen vernetzte MHC-Moleküle enthält.

Weiterhin können oligomerisierte Peptid-MHC-Komplexe durch Modifikation rekombinant hergestellter MHC-Moleküle erzeugt werden. So kann bei Herstellung der Vektoren für die Expression rekombinanter α- bzw. β-Ketten von MHC-Klasse II-Molekülen ein Gensegment, vorzugsweise jeweils am C-Terminus, einkloniert werden, das für ein Epitop codiert, das von einem Antikörper erkannt wird. Dieser Antikörper kann vom IgG-Typ, vorzugsweise aber vom IgM-Typ sein. Die renaturierten monomeren Peptid/MHC-Komplexe werden dann mit einem, das eingeführte Epitop erkennenden Antikörper inkubiert, so daß nicht-kovalent vernetzte Immunkomplexe, bestehend aus mehreren Antikörpern und mehreren Peptid-MHC-Komplexen, erzeugt werden. Die Einführung von DNA-Segmenten, die für ein Epitop codieren, in die für die α- bzw. β-Kette des MHC-Moleküls codierenden DNA-Fragmente kann mittels bekannter molekularbiologischer Techniken erfolgen, z.B. durch Insertion in Restriktionsstellen oder durch zielgerichtete Mutagenese.

Der erfindungsgemäße oligomerisierte Peptid-MHC-Komplex enthält vorzugsweise ein Peptid, das die Aminosäuresequenzen (I), (II), die in den Abbildungen 1 und 2 dargestellten Aminosäuresequenzen, Teilbereiche davon oder/und davon abgeleitete Aminosäuresequenzen umfaßt, oder ein Peptid-Derivat oder Peptidmimetikum davon. Der oligomerisierte Komplex kann vorzugsweise als diagnostisches oder therapeutisches Reagenz bei Typ I-Diabetes eingesetzt werden.

Die Erfindung betrifft somit auch eine pharmazeutische Zusammensetzung, die einen Peptid-MHC-Komplex als aktive Komponente gegebenenfalls in Kombination mit pharmazeutisch üblichen Zusatzstoffen enthält. Die Zusammensetzung kann weiterhin eine akzessorische stimulierende Komponente enthalten; z.B. Cytokine wie IL-2 und IL-4 oder/und das Oberflächenantigen B7 (Wyss-Coray et al., Eur. J. Immunol. 23 (1993) , 2175-2180; Freeman et al., Science 262 (1993), 909-911), das mit dem Oberflächenmolekül CD-28 auf einer T-Zelle binden kann. Die Anwesenheit der akzessorischen stimulierenden Komponente kann die therapeutische Wirkung der Zusammensetzung verbessern oder/und modifizieren.

Ein Gegenstand der vorliegenden Erfindung ist ferner die Verwendung einer pharmazeutischen Zusammensetzung, die einen Peptid-MHC-Komplex enthält zur Herstellung eines Mittels für die Diagnose von Erkrankungen oder einer Prädisposition für Erkrankungen, die das Immunsystem beeinflussen, oder für die Diagnose von Tumorerkrankungen oder einer Prädisposition für Tumorerkrankungen, insbesondere für die Diagnose von Autoimmunerkrankungen oder einer Prädisposition für Autoimmunerkrankungen, z.B. Diabetes Typ I oder Typ II, vorzugsweise Diabetes Typ I.

Analoge diagnostische Anwendungen sind jedoch auch bei anderen Autoimmunerkrankungen möglich. Beispiele derartiger Autoimmunerkrankungen sind Multiple Sklerose, wo reaktive T-Zellen gegen das Myelin Basic Protein oder das Proteolipid-Protein bestimmt werden können, rheumatoide Arthritis, wo reaktive T-Zellen gegen Kollagen Typ II, Cytokeratine und Hsp 65 bestimmt werden können, Basedow-Krankheit, wo reaktive T-Zellen gegen Thyroidperoxidase bestimmt werden können.

Allgemein ist die diagnostische Anwendung bei allen Erkrankungen möglich, die das Immunsystem beeinflussen, wie z.B. auch bei der Arteriosklerose. Hier wurde eine Assoziation der Krankheit mit einer Immunantwort gegen das Heat Shock Protein Hsp 65 nachgewiesen (Xu et al., Lancet 341, 8840 (1993), 255-259).

Noch eine weitere Anwendung ist der diagnostische Nachweis von T-Zellen, die gegen Tumorantigene reagieren. Beispiele hierfür sind T-Zellen gegen ein Melanom-assoziiertes Antigen MAGE 1, die aus Melanompatienten isoliert wurden (van der Bruggen et al., Science 254 (1991), 1643-1647). Der Nachweis dieser T-Zellen kann mit erfindungsgemäßen oligomerisierten Komplexen schon in einem Stadium erfolgen, in dem der Tumor aufgrund einer noch zu geringen Zellmasse mit herkömmlichen Methoden noch nicht nachweisbar ist. Ferner könnte der Nachweis von spezifisch reagierenden T-Zellen auch zur Verlaufskontrolle bei einer Anti-Tumorvakzinierung eingesetzt werden.

Ein weiterer Gegenstand der vorliegenden Erfindung ist somit ein Verfahren zur Bestimmung einer spezifischen T-Zell-Subpopulation, welches dadurch gekennzeichnet ist, daß man eine T-Zellen enthaltende Probe, die vorzugsweise aus einer Körperflüssigkeit, z.B. Vollblut, stammt, mit einem erfindungsgemäßen Komplex in Kontakt bringt und die Reaktion von T-Zellen mit dem Peptid oder Komplex bestimmt. Eine spezifische Reaktion von T-Zellen mit dem Komplex kann z.B. durch eine erhöhte T-Zellenproliferation nachgewiesen werden die sich durch den Einbau von Radioaktivität messen läßt. Andererseits kann die Reaktion von T-Zellen auch direkt durch Verwendung eines markierten Komplexes bestimmt werden. Bei dieser Ausführungsform wird der Komplex vorzugsweise mit einer daran gekoppelten Fluoreszenzmarkierungsgruppe verwendet. Die Auswertung kann beispielsweise durch FACS-Analyse erfolgen, wobei die T-Zellen mit einem ersten Fluoreszenzmarker, der an einen T-Zell-spezifischen Antikörper gekoppelt ist, und dann mit dem Peptid- MHC-Komplex, der mit einem zweiten Fluoreszenzmarker gekoppelt ist, in Kontakt gebracht werden und das Vorhandensein von doppelmarkierten Zellen durch fluorographische Analyse bestimmt wird. Auf diese Weise wird eine T-Zell-Subpopulation bestimmt, die durch ihre Reaktivität mit einem erfindungsgemäßen Peptid-MHC-Komplex charakterisiert ist. Aufgrund der geringen Konzentration der spezifischen T-Zell-Population im Blut erfolgt als erster Schritt des Verfahrens vorzugsweise eine Selektion auf präaktivierte T-Zellen, z.B. eine selektive Anreicherung von IL-2-Rezeptor-positiven T-Zellen durch Inkubation mit IL-2 oder/und durch Inkubation mit IL-2-Rezeptor-Antikörper und anschließende Separation der Antikörper-bindenden Zellen beispielsweise mit immunmagnetischen Methoden. Andererseits kann die Selektion auf präaktivierte Zellen erst nach dem Kontakt der T-Zellen mit dem Peptid oder dem Komplex erfolgen.

In einer Abwandlung dieses Verfahrens kann auch das Verhältnis von präaktivierten autoreaktiven T-Zellen, d.h. T-Zellen mit dem IL-2-Rezeptor als Oberflächenmarker, zu nicht-aktivierten autoreaktiven T-Zellen, d.h. T-Zellen ohne den IL-2-Rezeptor, bestimmt werden.

Dieses Verfahren kann insbesondere zur Diagnose von Typ I-Diabetes, aber auch bei anderen das Immunsystem beeinflussenden Erkrankungen bzw. zur Diagnose einer Prädisposition für derartige Erkrankungen angewendet werden.

Ein weiterer Gegenstand der vorliegenden Erfindung ist die Verwendung einer pharmazeutischen Zusammensetzung, die einen Peptid-MHC-Komplex enthält, zur Herstellung eines Mittels für die Therapie oder Prävention von Erkrankungen, die das Immunsystem beeinflussen. Zur therapeutischen Anwendung der erfindungsgemäßen Peptid-MHC-Komplexe können beispielsweise mit Toxinen gekoppelte Peptid-MHC-Komplexe verwendet werden, andererseits können aber auch Peptide als Bestandteile des Komplexes eingesetzt werden, die zwar eine Bindung an den T-Zellrezeptor ermöglichen, aber keine Aktivierung der T-Zelle hervorrufen, d.h. die also anergisierend wirken.

Die therapeutische Wirkung derartiger anergisierender Peptidanaloga beruht darauf, daß der T-Zellrezeptor (TCR) zur Aktivierung der T-Zelle mit einem Peptid wechselwirken muß, das von einem MHC-Antigen der Klasse I oder Klasse II präsentiert wird. Dabei sind insbesondere Aminosäuren in Ankerpositionen des Peptids für die Bindung an das MHC-Molekül verantwortlich, während andere Aminosäuren im Peptid zur Wechselwirkung mit dem TCR beitragen und somit eine T-Zellstimulation hervorrufen. Durch Aminosäuresubstitutionen in den Peptiden können nun Peptidanaloga hergestellt werden, die aufgrund des Vorhandenseins der Ankerpositionen noch an das MHC-Molekül binden, andererseits aber nur eine partielle oder keine T-Zellaktivierung hervorrufen (vgl. z.B. Sloan-Lancaster et al., Nature 363 (1993), 156-159). Z.B. können solche Peptidanaloga bewirken, daß die Expression bestimmter Oberflächenmoleküle hochreguliert wird (z.B. I12-Rezeptor, LFA-1), daß jedoch keine Proliferation oder Cytokin-Expression erfolgt. T-Zellen, die mit einem solchen Peptidanalogon in Wechselwirkung treten, gehen in einen sogenannten anergen Zustand über, d.h. sie können auch durch eine nachfolgende reguläre Stimulation mit einem Peptid nicht mehr proliferieren. Dieser anerge Zustand hält mindestens 7 Tage an und läßt sich deshalb therapeutisch bei der Behandlung einer Autoimmunerkrankung nutzen.

Ein weiterer therapeutischer Aspekt der vorliegenden Erfindung besteht darin, daß der Komplex aus Peptid und MHC-Molekül als Antigen verwendet werden kann. Ein derartiges Antigen kann dabei als Immunogen, d.h. als ein die Immunantwort stimulierendes Mittel oder als Tolerogen wirken, d.h. als ein Mittel, das eine Immuntoleranz hervorruft. Die Verwendung als Immunogen kann z.B. bei der Vakzinierung gegen Tumorantigene Verwendung finden. Statt den bisher zu diesem Zweck verwendeten ganzen Tumorzellen ist es möglich, daß von den T-Zellen erkannte tumorspezifische Peptide im Komplex mit dem entsprechenden MHC-Molekül, insbesondere in Form eines oligomerisierten Komplexes, zu injizieren, um eine T-Zellantwort gegen den Tumor zu erzeugen. Zur Erhöhung der Immunstimulation kann dieser Komplex auch in Kombination mit zusätzlichen stimulierenden Substanzen verabreicht werden. Zu diesem Zweck sind beispielsweise Cytokine, wie etwa IL2 oder IL4 geeignet, die gegebenenfalls und vorzugsweise kovalent mit dem erfindungsgemäßen Peptid-MHC-Komplex verknüpft sind. Eine weitere Möglichkeit ist die Assoziierung des Komplexes mit akzessorischen Komponenten für die T-Zellaktivierung, insbesondere mit für Antigen präsentierenden Zellen essentiellen Oberflächenmolekülen, z.B. dem Oberflächenmolekül B7.

Eine bevorzugte therapeutische Formulierung ist der Einbau von mit Peptid beladenen MHC-Molekülen in künstliche Vesikel, z.B. Lipidvesikel, die gegebenenfalls noch weitere membrangebundene Moleküle tragen können, wie z.B. B7 oder/und immobilisierte Cytokine.

Ein weiterer Gegenstand der vorliegenden Erfindung ist die Isolierung von T-Zellsubpopulationen, die mit einem erfindungsgemäßen Peptid-MHC-Komplex reagieren. Bei einem solchen Verfahren bringt man eine T-Zellen enthaltende Probe, die z.B. aus einer Körperflüssigkeit stammt, die einem Patienten vorher entnommen wurde, mit einem erfindungsgemäßen Peptid-MHC-Komplex in Kontakt, identifiziert die mit dem Komplex reagierenden T-Zellen und trennt sie gegebenenfalls von anderen T-Zellen ab. Auch hier kann vor oder/und nach dem Kontakt der T-Zellen mit dem Komplex vorzugsweise eine Selektion auf präaktivierte T-Zellen, d.h. T-Zellen mit dem IL-2-Rezeptor, erfolgen.

Bei einem solchen Verfahren kann man den Peptid-MHC-Komplex in immobilisierter Form auf einem Träger verwenden, wodurch die Abtrennung der positiv reagierenden T-Zell-Population von anderen T-Zellen vereinfacht wird. Aus den auf diese Weise isolierten T-Zell-Subpopulationen können durch Restimulation T-Zellinien angelegt werden. Diese autoreaktive T-Zellinien können dann zur Immunisierung von Patienten verwendet werden. Daher ist ein weiterer Gegenstand der Erfindung die Verwendung von nach dem erfindungsgemäßen Verfahren zur Isolierung einer spezifischen T-Zellsubpopulation isolierten T-Zellen oder Teilstrukturen davon zur Herstellung eines Medikaments zur Reinjektion in den Patienten, aus dem die T-Zellen oder Teilstrukturen davon ursprünglich stammen. Hierbei können die T-Zellen inaktivierte T-Zellen sein. Die T-Zellen können auch teilungsfähige T-Zellen sein.

Eine spezifische Immuntherapie des Typ I-Diabetes umfaßt zunächst die Isolierung von spezifischen T-Zellinien gegen ein Autoantigen, z.B. GAD 65 aus IDDM-Patienten. Dann erfolgt eine Bestimmung der Feinspezifität der T-Zellinien, d.h. die Identifizierung der autoreaktiven Peptide. Für die spätere Inokulation der Patienten werden solche T-Zellinien ausgewählt, die ein prädominantes Peptid erkennen, d.h. ein Peptid, gegen das mehrere der isolierten T-Zellinien reagieren. Insbesondere handelt es sich dabei um T-Zelllinien, welche ein Peptid mit den Aminosäuresequenzen (I) oder (II) erkennen.

Falls sich bei einem Patienten kein eindeutig prädominantes Peptid findet, müssen für die spätere Inokulation mehrere T-Zellinien gemischt werden. Die ausgewählten T-Zellklone werden vor der Inokulation nochmals mit Antigen-präsentierenden Zellen und den entsprechenden Peptiden stimuliert, um eine gute Expression von Aktivierungsmolekülen und insbesondere der T-Zellrezeptoren zu gewährleisten. Dann werden die T-Zellinien inaktiviert, z.B. durch Hitzebehandlung oder/und radioaktive Bestrahlung, vorzugsweise mit einer Dosis im Bereich von 4000 - 10000 rad, besonders bevorzugt ca. 8000 rad, und subkutan in einer Zellenzahl von vorzugsweise 10⁷ bis 5 x 10⁷ in den Patienten, aus dem sie gewonnen wurden, injiziert. Üblicherweise werden mindestens drei Injektionen über einen Zeitraum von 6 bis 12 Monaten verteilt.

Anschließend kann man die T-Zellantwort des Patienten auf das Inokulat testen. Hierzu isoliert man die peripheren Blutlymphozyten (PBLs) des Patienten, z.B. über Ficoll-Dichte-Gradientenzentrifugation, und testet die Proliferation gegen das Inokulat in einem Standard-Proliferationstest. Nach erfolgreich verlaufender Immunisierung sollte eine deutliche Proliferation der Patienten-PBLs gegen das Inokulat nachweisbar sein. Eine weitere Kontrolle des Immunisierungserfolgs kann durch Bestimmung der Frequenzen der GAD-reaktiven T-Zellen des Patienten im Verlauf der Immunisierung erfolgen. Dies kann z.B. nach dem Standardverfahren der Limiting Dilution mit autologen Stimulatorzellen erfolgen, die nach Inkubation mit GAD mit z.B. 4000 rad bestrahlt worden sein. Bei erfolgreich verlaufender Immunisierung nimmt die Frequenz der autoreaktiven T-Zellen deutlich ab.

Nach weiterer Eingrenzung der von den regulatorischen T-Zellen erkannten Oberflächenstrukturen auf den T-Zellen des Inokulates kann auch mit Teilstrukturen der regulatorischen T-Zellen immunisiert werden, z.B. mit Segmenten des T-Zellrezeptors.

Andererseits können bei einer Antitumorvakzinierung auch teilungsfähige T-Zellen reinjiziert werden, die zu einer aktiven Immunisierung des Patienten gegen Tumorzellen führen können.

Bei den diagnostischen und therapeutischen Verfahren zur Identifizierung bzw. Aktivierung/Inhibierung von spezifischen T-Zellsubpopulationen kann anstelle der erfindungsgemäßen Peptid-MHC-Moleküle auch ein anti-idiotypischer Antikörper verwendet werden, der die Wirkung des MHC-Peptid-Komplexes nachahmt. Derartige Antikörper können ohne weiteres erhalten werden, indem eine gegen ein bestimmtes Peptid spezifische T-Zellsubpopulation als Immunogen zur Erzeugung eines Antikörpers (z.B. in einer Maus) verwendet wird oder indem zuerst ein erster Antikörper gegen den MHC-Peptid-Komplex und dann ein anti-idiotypischer Antikörper gegen den ersten Antikörper erzeugt wird.

Ein Gegenstand der vorliegenden Erfindung ist somit auch ein Antikörper (erster Antikörper) gegen einen erfindungsgemäßen Komplex, erhältlich durch Immunisierung mit dem Komplex und Gewinnung eines durch Immunisierung erzeugten Antikörpers, vorzugsweise eines durch das Verfahren von Köhler und Milstein oder Weiterentwicklungen davon hergestellten monoklonalen Antikörpers.

Schließlich betrifft die Erfindung auch einen anti-idiotypischen Antikörper gegen den ersten Antikörper, erhältlich durch Immunisierung mit dem ersten Antikörper, der gegen den Komplex gerichtet ist, und Gewinnung eines durch die Immunisierung erzeugten anti-idiotypischen Antikörpers.

Noch ein weiterer Gegenstand der vorliegenden Erfindung ist eine T-Zelle, die mit einem erfindungsgemäßen Komplex aus Peptid und MHC-Molekül reagiert. Bevorzugte Beispiele sind T-Zellen, die von den T-Zellinien 6/7 (DSM ACC2172) oder 6/10 (DSM ACC2173) stammen oder eine äquivalente T-Zellrezeptor-Bindungsspezifität aufweisen, d.h. ein von einem MHC-Molekül präsentiertes Peptid oder Peptid-Derivat der Aminosäuresequenzen (I) oder/und (II) oder/und Teilbereichen dieser Aminosäuresequenzen erkennen.

Weiter soll die Erfindung durch die folgenden Beispiele in Verbindung mit den Abbildungen 1 bis 4 erläutert werden.
- Abb. 1: zeigt erfindungsgemäße autoreaktive Aminosäuresequenzen,
- Abb. 2: zeigt weitere erfindungsgemäße autoreaktive Aminosäuresequenzen,
- Abb. 3: zeigt das Ergebnis eines Proliferationsassays der T-Zellinie 6/7 mit Pepitdpools,
- Abb. 4: zeigt das Ergebnis eines Proliferationsassays der T-Zellinie 6/10 mit Peptidpools,
- Abb. 5: zeigt das Ergebnis eines Proliferationsassays mit der T-Zellinie 6/7 mit Einzelpeptiden aus Pool 7 und 11,
- Abb. 6: zeigt das Ergebnis eines Proliferationsassays mit der T-Zellinie 6/10 mit Einzelpeptiden aus Pool 7 und 11,

### BEISPIEL 1

### Etablierung von GAD-spezifischen T-Zellinien

### 1. Primärstimulation

Durch Ficoll-Dichtegradienten-Zentrifugation werden aus EDTA-Blut von Typ I-Diabetikern die peripheren Blut-Lymphozyten (PBLs) gewonnen. Die Zellen werden 2 mal in RPMI-Medium gewaschen und dann in einem Kulturmedium, bestehend aus RPMI 1640, 5 % Humanserum, 2 mM Glutamin und 100 U/ml Penicillin und 100 µg/ml Streptomycin, aufgenommen. Pro Napf einer 96 Napf Rundboden-Platte werden 100 µl Zellsuspension, entsprechend 100000 Zellen, eingesät. Danach erfolgt die Zugabe von Schweine-GAD (SW-GAD) in einer Endkonzentration von 2,5 µg/ml. Die Zellen werden 3 - 4 Tage im Blutschrank bei 37°C/7 % CO₂ inkubiert. Nach diesem Zeitraum erfolgt Zugabe von 100 µl IL-2 (30 U/ml). Nach weiteren 3 - 4 Tagen werden von allen Kulturansätzen 100 µl abgesaugt und wie-derum 100 µl IL-2 (30 U/ml) zugegeben. Dies wird alle 3 - 4 Tage wiederholt.

### 2. Restimulation

Am Tag 14 nach dem Beginn der Primärstimulation erfolgt die erste Restimulation. Hierfür wird im Vergleich zur Primärstimulation die doppelte Anzahl von autologen PBLs mittels Ficoll isoliert und in Kulturmedium auf eine Zellkonzentration von 2 x 10⁶/ml eingestellt. Eine Hälfte dieser Stimulatorzellen wird mit dem Antigen SW-GAD (Endkonzentration 5 *µ*g/ml) für 2 Stunden/37°C/7% CO₂ inkubiert (Antigen-Pulse). Die andere Hälfte wird unter gleichen Bedingungen ohne Antigen, nur mit Kulturmedium inkubiert. Anschließend werden alle Stimulatorzellen mit 4000 rad bestrahlt. Die Stimulatorzellen werden dann in 96 Napf Rundboden-Platten verteilt (je 100000 Zellen/Napf) und zwar so, daß immer ein Napf mit Antigen enthaltenden Stimulatorzellen benachbart zu einem Loch mit Stimulatorzellen ohne Antigen zu liegen kommt.

Anschließend erfolgt die Präparation der T-Zellen aus den Primärstimulationsansätzen. Hierfür werden die Überstände aus den Primärstimulationsansätzen abgesaugt und die Zellen in den Platten zweimal mit je 100 µl Waschmedium (Dulbeccos Modified Eagle Medium = DMEM) gewaschen. Dazwischen werden die Zellen in den Platten bei 400 g zentrifugiert. Anschließend werden die Zellen in je 100 µl Kulturmedium aufgenommen und je 50 µl auf zwei benachbarte Näpfe der Restimulations-Platte verteilt. Auf diese Weise werden die T-Zellen in einem Napf mit Antigen inkubiert und im benachbarten Napf ohne Antigen kann die Antigen-Spezifität der Restimulation kontrolliert werden.

Ab dem 2. oder 3. Tag nach dem Beginn der Restimulation kann die Proliferation mikroskopisch beurteilt werden. Dabei werden nur solche Mikrokultur-Pärchen als relevant angesehen, bei denen nur im Napf mit Antigen-Anwesenheit Proliferation erfolgt. Ab Tag 4 wird wiederum zu jedem Kultur-Napf 100 µl IL-2 (30 U/ml) zugegeben. Bis zum Tag 14 wird alle 3 - 4 Tage ca. 50 % des Kulturme-diums gegen IL-2 (30 U/ml) ausgetauscht.

Bei gutem Wachstum werden die Kulturen auf mehrere 96er Näpfe aufgeteilt. Bei späteren Restimulationen kann auch in größere Näpfe aufgeteilt werden. Alle 2 Wochen erfolgt eine erneute Restimulation nach der oben beschriebenen Methode. Ab der 3. Restimulation wird die Spezifität der Mikrokulturen in einem Proliferationstest ermittelt.

### 3. Proliferationstest mit SW-GAD

Alle Tests werden mindestens in Doppel-Ansätzen durchgeführt.

### a) Stimulator-Zellen:

Als Stimulatorzellen werden autologe PBLs oder in den HLA-Klasse II Antigenen identische PBLs eines normalen Spenders verwendet. Die PBLs werden wie unter Absatz 2. beschrieben mit Antigen vorinkubiert, mit 4000 rad bestrahlt und in 96 Napf-Platten verteilt (100000 Zellen/Loch) .

### b) T-Zellen

Die verwendeten T-Zellen stammen immer aus der Abschlußphase einer Restimulationsperiode. Sie werden 3 mal mit DMEM von Antigen und IL-2-freigewaschen und mit 6000 Zellen/96er Napf verteilt. Die auf diese Weise isolierten T-Zellinien 6/7 und 6/10 wurden bei der DSM unter den Nummern DSM ACC2172 bzw. DSM ACC2173 nach den Vorschriften des Budapester Vertrags hinterlegt.

Zusätzlich zu den Ansätzen mit SW-GAD werden Kontrollen ohne GAD inkubiert.

Nach 3 - 4 Tagen bei 37°C/7 % CO₂ erfolgt die Zugabe von 1 *µ*Ci ³H-Thymidin und weitere Inkubation für 16 - 20 Stunden. Danach erfolgt das Übertragen der Zellen auf einen Glasfaserfilter mittels eines Zell-Ernte Gerätes und die Bestimmung der eingebauten Radioaktivität im β-Zählgerät.

Tabelle 1 zeigt ein typisches Ergebnis eines Proliferationstests mit SW-GAD.

**Tabelle 1:**

| Ergebnisse des Proliferationstests der T-Zellinien 6/7 und 6/10 mit SW-GAD | | |
|---|---|---|
| Zell-linie | Kontrolle ohne Antigen | cpm SW-GAD |
| 6/7 | 129 | 9373 |
| 6/10 | 117 | 5222 |

### 4. Proliferationstest mit Peptiden, die aus der H-GAD Sequenz abgeleitet sind

T-Zellinien, die über mindestens 4 Restimulationsrunden expandiert wurden und mit SW-GAD im Proliferationstest reagierten, wurden zusätzlich mit überlappenden Peptiden der H-GAD getestet. Diese Experimente haben zum Ziel, die von den T-Zellen erkannten Epitope der H-GAD zu definieren. Dazu werden zunächst sich überlappende 20 mer Peptide der H-GAD synthetisiert (Überlappungsbe- reich 10 Aminosäuren, insgesamt 59 verschiedene Peptide).

Jeweils 4-5 dieser Peptide werden zu einem Pool vereinigt und in einer Endkonzentration von je 18 µg/ml zu den Stimulatorzellen gegeben (Präparation der Stimulatorzellen wie unter Abschnitt 3a beschrieben). Die weitere Behandlung dieser Stimulatorzellen wird wie unter Abschnitt 3a beschrieben, durchgeführt.

Danach erfolgt die Zugabe von 6000-20.000 T-Zellen pro Mikrokultur-Napf. Das weitere Verfahren ist analog dem unter Abschnitt 3b beschriebenen.

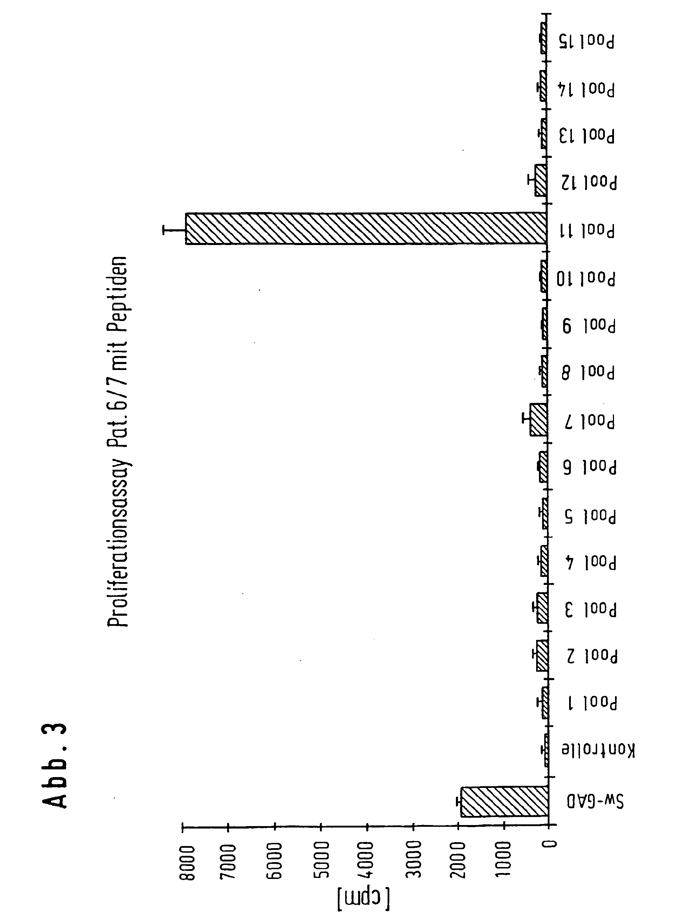

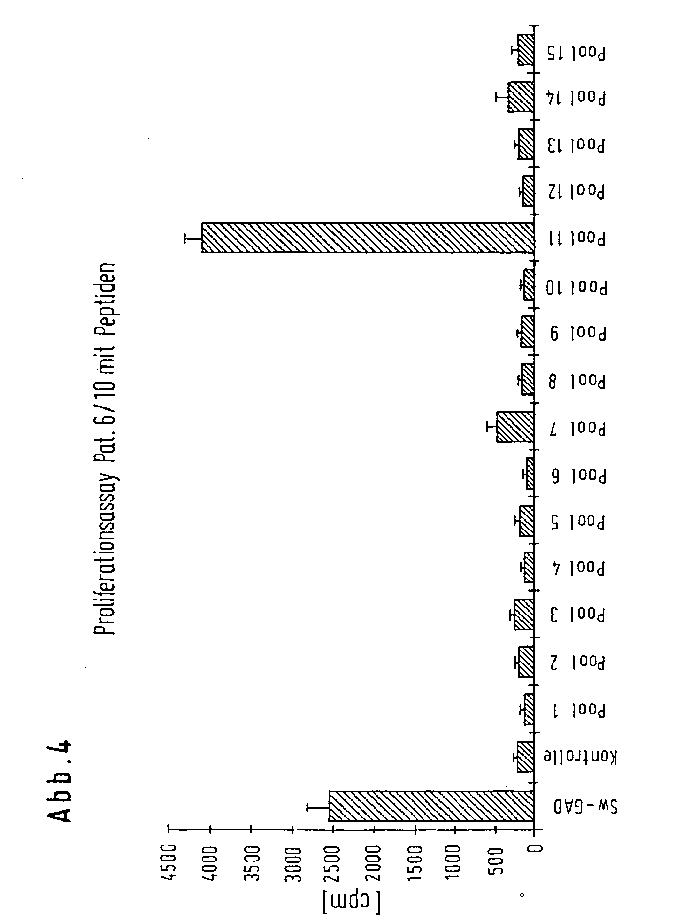

Die Abbildungen 3 und 4 zeigen Ergebnisse eines Proliferationsassays der T-Zellinien 6/7 und 6/10 unter Verwendung von Peptidpools der humanen GAD 65 kd. Beide T-Zellinien proliferieren stark mit Peptiden aus dem Pool 11. Eine geringere, aber signifikante Proliferation ist auch mit Pool 7 zu beobachten.

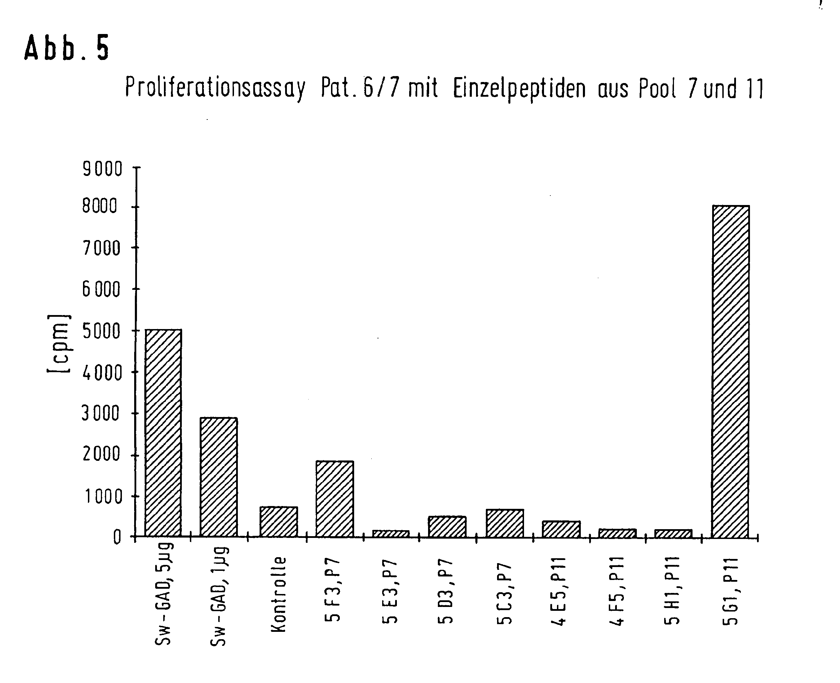

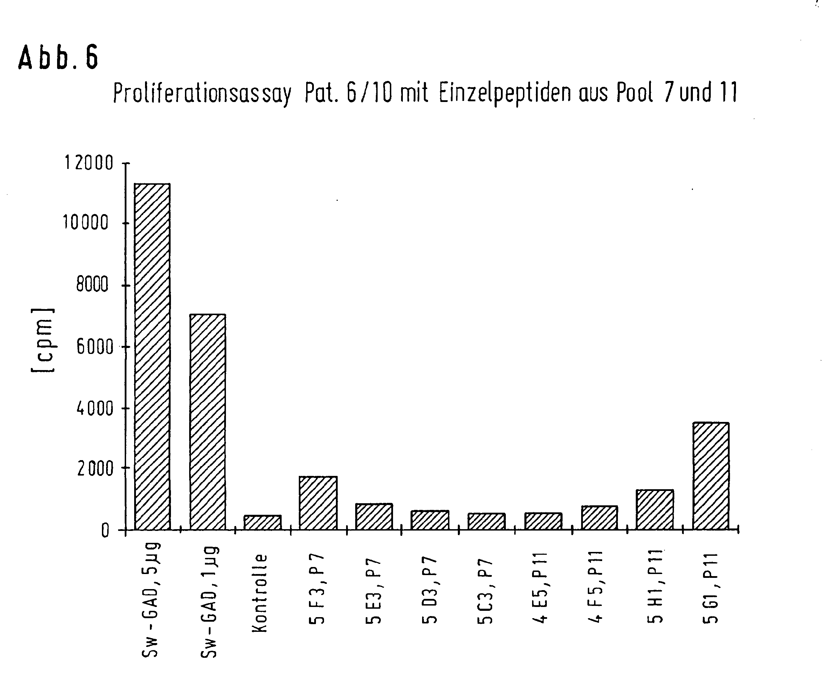

Die Abbildungen 5 und 6 zeigen Ergebnisse des Proliferationsassays der T-Zellinien 6/7 und 6/10 mit 10 µg/ml Einzelpeptiden aus den Pools 7 bzw. 11. Beide Zellinien zeigen eine signifikante Proliferation mit dem Peptid 5G1 (entsprechend den Aminosäuren 266-285 der humanen GAD 65) und eine geringere, jedoch signifikante Proliferation mit dem Peptid 5F3 (entsprechend den Aminosäuren 306-325 der humanen GAD 65).

### BEISPIEL 2

### Verfahren zur Isolierung und Bestimmung einer antigenspezifischen T-Zellsubpopulation

Da antigenspezifische, insbesondere gegen Auto-Antigene gerichtete T-Lymphozyten im peripheren Blut in sehr niedriger Zahl auftreten (erwartete Frequenz 10⁻⁵ - 10⁻⁶), bietet sich an, die in vivo präaktivierten T-Zellen über einen Selektionsschritt zunächst anzureichern.

Dies kann durch zwei Verfahren erzielt werden:

### 1. Expansion der in vivo präaktivierten T-Zellen durch Inkubation mit IL-2

Dazu werden PBLs mittels Ficoll-Dichtegradienten-Zentrifugation isoliert und in Zellkultur-Medium mit IL-2 (RPMI 1640/5 % Humanserium/30 U/ml IL-2) auf 2 x 10⁶ Zellen/ml eingestellt. Die Zellen werden in 200 µl Aliquoten auf 48 Napfplatten verteilt und 7 Tage inkubiert. Nach 4 Tagen wird zusätzlich noch einmal IL-2 zugegeben. Da präaktivierte T-Zellen den hochaffinen IL-2 Rezeptor exprimieren, proliferieren selektiv die in vivo präaktivierten T-Zellen während diesesr Stimulationsperiode und reichern sich in der Primärkultur an. Nach Abschluß der Stimulationsperiode werden die Zellen in den einzelnen Näpfen gewaschen, gezählt und in einem Proliferationstest verwendet.

### 2. Anreicherung von in vivo präaktivierten T-Zellen durch immunmagnetische Separation

Hierzu werden die Ficoll- isolierten PBLs mit monoklonalen Antikörpern gegen den hochaffinen IL-2 Rezeptor inkubiert (7 x 10⁶ PBLs/ml; 10 µg/ml anti IL-2 Rezeptor Antikörper (Boehringer Mannheim); 30 min bei 4°C). Anschließend wird die Zellsuspension zweimal mit eiskaltem RPMI/10 % Humanserum (HS) gewaschen (400 g/10 min) und dann die Suspension auf eine Zelldichte von 1 - 3 x 10⁷/ml eingestelt. Dazu werden mit Schaf-anti Maus-Antikörper gekoppelte Dynabeads M-280 der Firma Dynal gegeben (Verhältnis Dynabeads zu Zielzellen ca. 10 - 15). Die Suspension wird bei 4°C auf einem Roller sehr langsam bewegt. Danach wird die Suspension mit RPMI/10 % HS 10-fach verdünnt und für 1 - 2 Minuten in den zuvor gekühlten Magnetpartikel-Konzentrierer (MPC) gestellt. Nachdem die rosettierten IL-2 Rezeptor tragenden T-Zellen vom Magneten immobilisiert sind, wird der Überstand abgesaugt, das Inkubationsgefäß aus dem MPC entfernt und die verbleibenden Zellen mit RPMI/10 % HS resuspendiert. Die Abtrennung der Zielzellen erfolgt noch einmal im MPC. Dieser Waschschritt wird noch einmal wiederholt. Anschließend werden die separierten Zellen in Kulturmedium resuspendiert und die Zellzahl auf 1 x 10⁷/ml eingestellt. Die Magnetobeads werden nach bekannten Verfahren über die Detacher Antikörper entfernt.

Die über die Verfahren nach 2.1 oder 2.2 angereicherten präaktivierten Zellen werden anschließend auf Reaktivität gegen die Auto-Antigen Peptide bzw. gegen einen Peptid/MHC-Komplex getestet.

Auch hier bieten sich mehrere Verfahren an:

### 3. Proliferationstest mit bestrahlten Stimulatorzellen und Peptiden als Antigene

Zunächst präpariert man autologe Stimulatorzellen aus Ficollisolierten PBLs. Die Stimulatorzellen werden auf eine Konzentration von 10⁶ Zellen/ml in Zellkulturmedium eingestellt und mit den Peptiden (Endkonzentration 10 µg/ml) für 2 Stunden/37°C/ 7 % CO₂ inkubiert. Danach werden die Stimulatorzellen mit 4000 rad bestrahlt und anschließend in einer Zellzahl von 100000 Zellen/Napf in einer 96 Napf-Rundbodenplatte verteilt.

Dazu gibt man je 100000 der aus 2.1 oder 2.2 gewonnenen in vivo präaktivierten T-Zellen und inkubiert für 4 Tage/37°C/7 % CO₂. Dann wird die Hälfte des Ansatzvolumens gegen IL-2 (30 U/ml) ausgetauscht . Dies geschieht nach weiteren 4 Tagen noch einmal.

Am Tag 12 nach dem Start der Mikrokulturen wird der eigentliche Proliferationstest durchgeführt. Dazu werden zuerst die Mikrokulturen zweimal mit DMEM gewaschen, um Antigen und IL-2 zu entfernen. Jede Kultur wird in vier Aliquots aufgeteilt und in Duplikaten in der Anwesenheit von 100000 autologen, bestrahlten PBLs, jeweils mit oder ohne Peptid-Pulse, für 3 Tage inkubiert (37°C/7 % CO₂). Nach dieser Zeit wird 3H-Thymidin zugegeben und nach weiteren 16 - 20 Stunden die eingebaute Radioaktivität bestimmt.

### 4. Direkter Nachweis der Autoantigen-reaktiven T-Zellen durch Markierung über einen oligomerisierten Peptid/HLA-Komplex

Hierfür verwendet man die nach der Methode 2.2 angereichert in vivo präaktivierten T-Zellen. Die Zellen werden in RPMI/10 % HS auf eine Konzentration von 10⁶/ml eingestellt und mit dem oligomerisierten, mit einer Fluoreszenz-Markierung versehenen HLA-Peptid-Komplex bei 4°C für 30 min inkubiert. Anschließend werden die Zellen mit eiskaltem Zellkultur-Medium zweimal gewaschen. Die Analyse der Fluoreszenz-markierten Zellpopulation erfolgt im Durchflußcytometer.

### Beispiel 3

### Identifizierung von MHC-Molekülen, welche ein definiertes autoreaktives Peptid präsentieren

Hierzu werden zunächst die Peptide mit ¹²⁵J markiert, z.B. nach der Methode von Bolton und Hunter (Bolton, A.E. and Hunter, W.M., Biochem. J. 133 (1993), 529-531). Dann werden 2 - 5 x 10⁶ PBLs des Spenders mit dem zu untersuchenden MHC-Typ in Zellkulturmedium mit ¹²⁵J-markierten Peptiden (2 - 10 µM) für 4 Stunden bei 37°C inkubiert. Nach dem Waschen der Zellen werden diese in einem Lysepuffer, bestehend aus 0,5 % NP 40; 0,5 % Mega9; 150 mM NaCl; 5 mM EDTA; 50 mM Tris pH 7,5; 2 mM Phenylmethylsulfonylfluorid) lysiert. Aus der Mischung werden die MHC-Moleküle durch an Protein A-Sepharose gebundene Framework-spezifische monoklonale Antikörper (z.B. im Falle von HLA-DR mit dem monoklonalen Antikörper L243 (ATCC HB 55)) immunpräzipitiert und die an die Protein A-Sepharose gebundene Radioaktivität im Gamma-Counter bestimmt.

### BEISPIEL 4

### Bestimmung des Subtyps von MHC-Molekülen, die den T-Zellinien 6/7 (DSM ACC2172) und 6/10 (DSM ACC2173) autoreaktive Peptide präsentieren

Die Versuchsdurchführung erfolgte analog dem Beispiel 1.3. Als Antigen-repräsentierende Zellen wurden allerdings keine autologen PBLs verwendet, sondern PBLs von heterologen Donoren, welche nicht komplett, sondern nur in definierten MHC-Allelen mit den MHC-Molekülen des Donors, aus dem auch die T-Zellinien entwickelt wurden, übereinstimmen. Die Proliferationstests wurden unter Verwendung der autoantigenen Peptide 5G1 (entsprechend den Aminosäuren 266-285 der humanen GAD65) und 5F3 (entsprechend den Aminosäuren 306-325 der humanen GAD65) durchgeführt.

Die Tabelle 3 zeigt das Ergebnis eines solchen Testansatzes. Die T-Zellinien 6/7 und 6/10 proliferieren mit beiden Peptiden in Anwesenheit des DR B1-Allels 0401. Eine Variation der DQ A1- bzw. DQ Bi-Allele ist ohne Einfluß auf die Stimulierbarkeit der T-Zellinien. Die T-Zellinie 6/7 erkennt das Peptid 5G1 zusätzlich in Assoziation mit den Allelen DR B1 0101 oder/und 1601.

T-Zell-Proliferation nach Stimulation mit den Peptiden 5G1 und 5F3 unter Verwendung von PBLs mit verschiedenen Haplotypen als Antigen-präsentierende Zellen

**Tabelle 3**

| Spender | Haplotyp der APC DR B1* DQ A1*DQ B1* | | | Identität der Allele mit den Allelen des Spenders der TCL | TCL 6/7 cpm | | TCL 6/10 cpm | |
|---|---|---|---|---|---|---|---|---|
| | | | | | +Peptid | SI | +Peptid | SI |
| A.K. | 0301 | 0501 | 0201 | DR: 2 Allele ident. | 5G1 | 55,0 | 5G1 | 6,0 |
| | 0401 | 0301 | 0302 | DQ: 4 Allele ident. | 5F3 | 3,8 | 5F3 | 3,8 |
| G.H. | 0301 | 0501 | 0201 | DR: 1 Allel ident. | 5G1 | 0,9 | 5G1 | 1,5 |
| | | | | 1 Allel nicht ident. | 5F3 | 0,6 | 5F3 | 1,5 |
| | 0404 | 0301 | 0302 | DQ: 4 Allele ident. | | | | |
| G.E. | 1302 | 0102 | 0604 | DR: 1 Allel ident. | 5G1 | 67,8 | 5G1 | 22,6 |
| | | | | 1 Allel nicht ident. | 5F3 | 7,0 | 5F3 | 6,5 |
| | 0401 | 0301 | 0302 | DQ: 2 Allele ident. | | | | |
| | | | | 2 Allele nicht ident. | | | | |
| 19 | 0301 | 0501 | 0301 | DR: 2 Allele ident. | 5G1 | 45,7 | 5G1 | 8,5 |
| | 0401 | 0201 | 0301 | DQ: 1 Allel ident. 3 Allele nicht ident. | 5F3 | 3,1 | 5F3 | 2,8 |
| D.J. | 0101 | 0101 | 0501 | DR: 2 Allele nicht ident. | 5G1 | 28,6 | 5G1 | 2,2 |
| | 1601 | 0102 | 0502 | DQ: 4 Allele nicht ident. | 5F3 | 1,2 | 5F3 | 1,4 |

| | | | | | | | | |
|---|---|---|---|---|---|---|---|---|
| TCL = T-Zellinie APC = Antigen-präsentierende Zellen SI = Stimulationsindex: cpm in Anwesenheit des Peptides dividiert durch cpm ohne Peptid. | | | | | | | | |

### BEISPIEL 5

### Proliferationstest mit Varianten des Peptids 5G1 unter Verwendung der T-Zellinie 6/10

Um die Kernstruktur des stimulierenden Peptids 5G1 aufzuklären, wurden Proliferationstests mit verschiedenen Varianten dieses Peptids unter Verwendung der T-Zellinie 6/10 durchgeführt (siehe Tabelle 4).

Ein Test mit einer ersten Serie von 20 mer-Varianten sollte entschlüsseln, ob an die 5G1-Struktur am C- bzw. N-Terminus angrenzende Aminosäuren eine Rolle bei der Erkennung durch die T-Zellinie 6/10 spielen. Wie die Stimulationsindices zeigen, bringt eine Verschiebung des 20mer Peptids in Richtung C-Terminus keinen Gewinn an Proliferationsaktivität. Eine Verschiebung um 6 Aminosäuren in Richtung N-Terminus führt zu einem Verlust an Stimulationskapazität.

Ein Test mit einer zweiten Serie von Peptidvarianten untersucht den Einfluß einer Verkürzung am C-Terminus. Aus dieser Serie von Experimenten geht hervor, daß die C-terminalen Aminosäurereste Histidin (H) und Phenylalanin (F) für die Stimulationskapazität bedeutsam sind.

Ein Test mit einer dritten Serie von Peptidvarianten hatte das Ziel, den N-Terminus des minimalen stimulationsaktiven Peptids zu definieren. Entfernt man von einem 18mer mit intaktem C-Terminus die Aminosäuren Leucin (L) und Prolin (P), so kommt es zu einem starken Abfall des Stimulationsindex. Der N-Terminus ist somit durch L und P definiert. Eine weitere Verkürzung um H und F am C-Terminus führt ebenfalls zu einem Verlust an Stimulationsaktivität. Somit ist für die T-Zellinie 6/10 das minimale, noch stimulierende Peptid ein 14mer der Sequenz LPRLIAFTSEHSHF.

**Tabelle 4**

| | Reaktion von TCL 6/10 mit Varianten des Peptids 5G1 zur Identifizierung der minimalen, noch stimulationsaktiven Peptidstruktur | |
|---|---|---|
| | Peptid-Varianten von 5G1 | Stimulations-index |
| 5G1 | GMAALPRLIAFTSEHSHFSL | 5,4 |
| | ALPRLIAFTSEHSHFSLKKG | 3,0 |
| | RLIAFTSEHSHFSLKKGAAA | 3,2 |
| PEVKEKGMAALPRLIAFTSE | | 0,6 |
| | | |
| | AALPRLIAFTSEHSHFSL | 4,5 |
| | AALPRLIAFTSEHSHF | 2,9 |
| | AALPRLIAFTSEHS | 0,7 |
| | AALPRLIAFTSE | 0,6 |
| | GMAALPRLIAFTSE | 0,8 |
| | GMAALPRLIAFT | 1,0 |
| | | |
| | LPRLIAFTSEHSHFSLKK | 3,2 |
| | RLIAFTSEHSHFSL | 1,4 |
| | LPRLIAFTSEHSHF | 4,6 |
| | LPRLIAFTSEHS | 0,4 |

## Patentansprüche

1. Komplex, der mindestens ein Peptid oder Peptid-Derivat gebunden an ein MHC-Molekül oder ein peptidbindendes Derivat eines MHC-Moleküls umfasst, wobei das Peptid oder Peptid-Derivat eine maximale Länge von 100 Aminosäuren aufweist und umfasst:
(a) eine der in SEQ ID NO. 1 bis SEQ ID NO. 23 gezeigten Aminosäuresequenzen,
(b) Teilbereiche der in SEQ ID NO. 1 bis SEQ ID NO. 23 gezeigten Aminosäuresequenzen mit einer Länge von mindestens 6 Aminosäuren, die eine im Wesentlichen äquivalente Spezifität oder/und Affinität der Bindung an humane MHC-Moleküle, wie die Aminosäuresequenzen aus (a) zeigen, oder/und
(c) von den in SEQ ID NO. 1 bis SEQ ID NO. 23 gezeigten Aminosäuresequenzen abgeleitete Aminosäuresequenzen, die eine im Wesentlichen äquivalente Spezifität oder/und Affinität der Bindung an humane MHC-Moleküle, wie die Aminosäuresequenzen aus (a) zeigen, wobei die von den in SEQ ID NO. 1 bis SEQ ID NO. 23 gezeigten Aminosäuresequenzen abgeleiteten Aminosäuresequenzen eine Sequenzhomologie von mindestens 30 % zu den Ausgangsaminosäuresequenzen aufweisen.

2. Komplex nach Anspruch 1, wobei das Peptid oder Peptid-Derivat umfasst:
(a) die in SEQ ID NO. 1 gezeigte Aminosäuresequenz,
(b) die in SEQ ID NO. 2 gezeigte Aminosäuresequenz,
(c) Teilbereiche der in SEQ ID NO. 1 oder/und SEQ ID NO. 2 gezeigten Aminosäuresequenzen, die eine im Wesentlichen äquivalente Spezifität oder/und Affinität der Bindung an humane MHC-Moleküle, wie die Aminosäuresequenzen aus (a) oder/und (b) zeigen, oder/und
(d) von den in SEQ ID NO. 1 oder/und SEQ ID NO. 2 gezeigten Aminosäuresequenzen abgeleitete Aminosäuresequenzen, die eine im Wesentlichen äquivalente Spezifität oder/und Affinität der Bindung an humane MHC-Moleküle, wie die Aminosäuresequenzen aus (a) oder/und (b) zeigen, wobei die von den in SEQ ID NO. 1 oder/und SEQ ID NO. 2 gezeigten Aminosäuresequenzen abgeleiteten Aminosäuresequenzen eine Sequenzhomologie von mindestens 30 % zu den Ausgangsaminosäuresequenzen aufweisen.

3. Komplex nach Anspruch 1 oder 2,
**dadurch gekennzeichnet,**
**dass** das Peptid oder Peptid-Derivat eine Länge von mindestens 8 Aminosäuren aufweist.

4. Komplex nach einem der Ansprüche 1 bis 3,
**dadurch gekennzeichnet,**
**dass** das Peptid oder Peptid-Derivat eine Länge von mindestens 10 Aminosäuren aufweist.

5. Komplex nach einem der Ansprüche 1 bis 3,
**dadurch gekennzeichnet,**
**dass** das Peptid oder Peptid-Derivat eine Länge von bis zu 25 Aminosäuren aufweist.

6. Komplex nach einem der Ansprüche 1 bis 5,
**dadurch gekennzeichnet,**
**dass** das Peptid oder Peptid-Derivat eine Markierungsgruppe trägt.

7. Komplex, der mindestens ein Peptidmimetikum eines Peptids oder Peptid-Derivats, wie in Anspruch 1 oder 2 definiert, umfasst, das an ein MHC-Molekül oder ein peptidbindendes Derivat eines MHC-Moleküls gebunden ist,
**dadurch gekennzeichnet,**
**dass** das Peptidmimetikum eine im Wesentlichen äquivalente Spezifität oder/und Affinität der Bindung an humane MHC-Moleküle wie ein Peptid oder Peptid-Derivat nach einem der Ansprüche 1 bis 6 aufweist.

8. Komplex nach einem der Ansprüche 1 bis 7,
**dadurch gekennzeichnet,**
**dass** er ein MHC-Klasse II-Molekül oder ein peptidbindendes Derivat davon umfasst.

9. Komplex nach Anspruch 8,
**dadurch gekennzeichnet,**
**dass** das MHC-Klasse II-Molekül den Typ DR1, DR2, DR3 oder DR4 aufweist.

10. Komplex nach Anspruch 9,
**dadurch gekennzeichnet,**
**dass** das MHC-Klasse II-Molekül den Subtyp DR B1 0101, DR B1 0301, DR B1 0401, DR B1 0402, DR B1 0404 oder DR B1 1601 aufweist.

11. Komplex nach Anspruch 10,
**dadurch gekennzeichnet,**
**dass** das MHC-Klasse II-Molekül den Subtyp DR B1 0101 oder DR B1 0401 aufweist.

12. Komplex nach einem der Ansprüche 1 bis 11,
**dadurch gekennzeichnet,**
**dass** er ein rekombinantes MHC-Molekül oder ein peptidbindendes Derivat davon umfasst.

13. Komplex nach Anspruch 12,
**dadurch gekennzeichnet,**
**dass** er ein lösliches peptidbindendes Derivat eines MHC-Moleküls umfasst.

14. Komplex nach einem der Ansprüche 1 bis 13,
**dadurch gekennzeichnet,**
**dass** er eine Markierungsgruppe trägt.

15. Oligomerisierter Peptid-MHC-Molekül-Komplex nach einem der Ansprüche 1 bis 14, der mindestens 2 MHC-Moleküle oder MHC-Molekülderivate, die über kovalente oder nicht-kovalente Wechselwirkungen assoziiert sind, enthält.

16. Oligomerisierter Komplex nach Anspruch 15,
**dadurch gekennzeichnet,**
**dass** er durch chemische Kopplungsreagenzien quervernetzte Peptid-MHC-Molekül=Komplexe enthält.

17. Oligomerisierter Komplex nach Anspruch 15,
**dadurch gekennzeichnet,**
**dass** er durch eine oligomerisierte Peptidkomponente mit mehreren MHC-bindenden Bereichen vernetzte MHC-Moleküle oder MHC-Molekülderivate enthält.

18. Oligomerisierter Komplex nach Anspruch 15,
**dadurch gekennzeichnet,**
**dass** er durch Antikörper vernetzte Peptid-MHC-Molekül-Komplexe enthält.

19. Oligomerisierter Komplex nach einem der Ansprüche 15 bis 18,
**dadurch gekennzeichnet,**
**dass** er MHC-Moleküle wie in einem der Ansprüche 8 bis 13 definiert enthält.

20. Pharmazeutische Zusammensetzung,
**dadurch gekennzeichnet,**
**dass** sie einen Komplex nach einem der Ansprüche 1 bis 19 als aktive Komponente gegebenenfalls in Kombination mit pharmazeutisch üblichen Zusatzstoffen enthält.

21. Pharmazeutische Zusammensetzung nach Anspruch 20,
**dadurch gekennzeichnet,**
**dass** sie weiterhin eine akzessorische stimulierende Komponente umfasst.

22. Pharmazeutische Zusammensetzung nach Anspruch 21,
**dadurch gekennzeichnet,**
**dass** die akzessorische stimulierende Komponente ausgewählt ist aus Cytokinen oder/und dem Oberflächenantigen B7.

23. Verwendung einer pharmazeutischen Zusammensetzung nach einem der Ansprüche 20 bis 22 zur Herstellung eines Mittels für die Diagnose von Erkrankungen oder einer Prädisposition für Erkrankungen, die das Immunsystem beeinflussen, oder von Tumorerkrankungen oder einer Prädisposition für Tumorerkrankungen.

24. Verwendung nach Anspruch 23 zur Herstellung eines Mittels für die Diagnose von Autoimmunerkrankungen oder einer Prädisposition für Autoimmunerkrankungen.

25. Verwendung nach Anspruch 23 oder 24 zur Herstellung eines Mittels für die Diagnose von Diabetes oder einer Prädisposition für Diabetes.

26. Verfahren zur Bestimmung einer spezifischen T-Zell-Subpopulation,
**dadurch gekennzeichnet,**
**dass** man eine T-Zellen enthaltende Probe mit einem Komplex nach einem der Ansprüche 1 bis 19 in Kontakt bringt und die Reaktion von T-Zellen in der Probe mit dem Komplex bestimmt.

27. Verfahren nach Anspruch 26,
**dadurch gekennzeichnet,**
**dass** man die Reaktion der T-Zellen mit einem fluoreszenzmarkierten Komplex durch FACS-Analyse bestimmt.

28. Verfahren nach Anspruch 26 oder 27,
**dadurch gekennzeichnet,**
**dass** man vor und/oder nach dem Kontakt der T-Zellen mit dem Komplex eine Selektion auf präaktivierte T-Zellen durchführt.

29. Verwendung einer pharmazeutischen Zusammensetzung nach einem der Ansprüche 20 bis 22 zur Herstellung eines Mittels für die Therapie oder Prävention von Erkrankungen, die das Immunsystem beeinflussen.

30. Verwendung nach Anspruch 29 zur Herstellung eines Mittels für die Therapie oder Prävention von Autoimmunerkrankungen.

31. Verwendung nach Anspruch 29 oder 30 zur Herstellung eines Mittels für die Therapie oder Prävention von Diabetes.

32. Verwendung eines Komplexes nach einem der Ansprüche 1 bis 19 zur Herstellung eines Antigens, insbesondere eines lmmunogens oder Tolerogens.

33. Verfahren zur Isolierung einer spezifischen T-Zell-Subpopulation,
**dadurch gekennzeichnet,**
**dass** man eine T-Zellen enthaltende Probe mit einem Komplex nach einem der Ansprüche 1 bis 19 in Kontakt bringt, die mit dem Peptid oder Komplex reagierenden T-Zellen identifiziert und gegebenenfalls von anderen T-Zellen abtrennt.

34. Verfahren nach Anspruch 33,
**dadurch gekennzeichnet,**
**dass** man vor oder/und nach dem Kontakt der T-Zellen mit dem Komplex eine Selektion auf präaktivierte T-Zellen durchführt.

35. Verwendung von nach dem Verfahren gemäß Anspruch 33 isolierten T-Zellen oder Teilstrukturen davon zur Herstellung eines Antigens.

36. Verwendung von nach dem Verfahren gemäß Anspruch 33 isolierten T-Zellen oder Teilstrukturen davon zur Herstellung eines Medikaments zur Reinjektion in den Patienten, aus dem die T-Zellen oder Teilstrukturen davon ursprünglich stammen.

37. Verwendung nach Anspruch 36,
**dadurch gekennzeichnet,**
**dass** die T-Zellen inaktivierte T-Zellen sind.

38. Verwendung nach Anspruch 36,
**dadurch gekennzeichnet,**
**dass** die T-Zellen teilungsfähige T-Zellen sind.

39. Antikörper gegen einen Komplex nach einem der Ansprüche 1 bis 19, erhältlich durch Immunisierung mit dem Komplex und Gewinnung eines durch die Immunisierung erzeugten Antikörpers.

40. Anti-idiotypischer Antikörper gegen einen Antikörper nach Anspruch 39, erhältlich durch Immunisierung mit dem Antikörper gegen den Komplex und Gewinnung eines durch die Immunisierung erzeugten antiidiotypischen Antikörpers.

41. T-Zelle, die mit einem Komplex nach einem der Ansprüche 1 bis 19 reagiert.

42. T-Zelle nach Anspruch 41, die von der T-Zellinie 6/7 (DSM ACC2172) stammt oder eine äquivalente T-Zellrezeptor-Bindungsspezifität aufweist.

43. T-Zelle nach Anspruch 41, die von der T-Zellinie 6/10 (DSM ACC2173) stammt oder eine äquivalente T-Zellrezeptor-Bindungsspezifität aufweist.

## Claims

1. A complex which comprises at least one peptide or peptide-derivative bound to an MHC molecule or a peptide-binding derivative of an MHC molecule, the peptide or peptide derivative having a maximum length of 100 amino acids and comprising:
(a) one of the amino acid sequences shown in SEQ ID no. 1 to SEQ ID no. 23,
(b) subdomains of the amino acid sequences shown in SEQ-ID no. 1 to SEQ ID no. 23 with a length of at least 6 amino acids, which exhibit a substantially equivalent specificity and/or affinity for binding to human MHC molecules as the amino acid sequences from (a), and/or
(c) amino acid sequences derived from the amino acid sequences shown in SEQ ID no. 1 to SEQ ID no. 23 which exhibit a substantially equivalent specificity and/or affinity for binding to human MHC molecules as the amino acid sequences from (a), the amino acid sequences derived from the amino acid sequences shown in SEQ ID no. 1 to SEQ ID no. 23 having an at least 30% sequence homology to the initial amino acid sequences.

2. A complex according to claim 1, the peptide or peptide-derivative comprising:
(a) the amino acid sequence shown in SEQ ID no. 1,
(b) the amino acid sequence shown in SEQ ID no. 2,
(c) subdomains of the amino acid sequences shown in SEQ ID no. 1 and/or SEQ ID no. 2 which exhibit a substantially equivalent specificity and/or affinity for binding to human MHC molecules as the amino acid sequences from (a) and/or (b), and/or
(d) amino acid sequences derived from the amino acid sequences shown in SEQ ID no. 1 and/or SEQ ID no. 2 which exhibit a substantially equivalent specificity and/or affinity for binding to human MHC molecules as the amino acid sequences from
(a) and/or (b), the amino acid sequences derived from the amino acid sequences shown in SEQ ID no. 1 and/or SEQ ID no. 2 having an at least 30% sequence homology to the initial amino acid sequences.

3. A complex according to claim 1 or claim 2,
**characterised in that**
the peptide or peptide-derivative has a length of at least 8 amino acids.

4. A complex according to any one of claims 1 to 3,
**characterised in that**
the peptide or peptide-derivative has a length of at least 10 amino acids.

5. A complex according to any one of claims 1 to 3,
**characterised in that**
the peptide or peptide-derivative has a length of up to 25 amino acids.

6. A complex according to any one of claims 1 to 5,
**characterised in that**
the peptide or peptide-derivative bears a labelling group.

7. A complex which comprises at least one peptide mimetic of a peptide or peptide derivative, as defined in claim 1 or claim 2, which is bound to an MHC molecule or a peptide-binding derivative of an MHC molecule,
**characterised in that**
the peptide mimetic has a substantially equivalent specificity and/or affinity for binding to human MHC molecules as a peptide or peptide derivative according to any one of claims 1 to 6.

8. A complex according to any one of claims 1 to 7,
**characterised in that**
it comprises an MHC class II molecule or a peptide-binding derivative thereof.

9. A complex according to claim 8,
**characterised in that**
the MHC class II molecule is of type DR1, DR2, DR3 or DR4.

10. A complex according to claim 9,
**characterised in that**
the MHC class II molecule is of subtype DR B1 0101, DR B1 0301, DR B1 0401, DR B1 0402, DR B1 0404 or DR B1 1601.

11. A complex according to claim 10,
**characterised in that**
the MHC class II molecule is of subtype DR B1 0101 or DR B1 0401.

12. A complex according to any one of claims 1 to 11,
**characterised in that**
it comprises a recombinant MHC molecule or a peptide-binding derivative thereof.

13. A complex according to claim 12,
**characterised in that**
it comprises a soluble peptide-binding derivative of an MHC molecule.

14. A complex according to any one of claims 1 to 13,
**characterised in that**
it bears a labelling group.

15. An oligomerised peptide-MHC molecule complex according to any one of claims 1 to 14 which contains at least 2 MHC molecules or MHC molecule derivatives which are associated by covalent or non-covalent interaction.

16. An oligomerised complex according to claim 15,
**characterised in that**
it contains peptide-MHC molecule complexes crosslinked by chemical coupling reagents.

17. An oligomerised complex according to claim 15,
**characterised in that**
it contains MHC molecules or MHC molecule derivatives crosslinked by an oligomerised peptide component having two or more MHC-binding domains.

18. An oligomerised complex according to claim 15,
**characterised in that**
it contains peptide-MHC molecule complexes crosslinked by antibodies.

19. An oligomerised complex according to any one of claims 15 to 18,
**characterised in that**
it contains MHC molecules as defined in any one of claims 8 to 13.

20. A pharmaceutical composition,
**characterised in that**
it contains a complex according to any one of claims 1 to 19 as active component, optionally in combination with pharmaceutically conventional additives.

21. A pharmaceutical composition according to claim 20,
**characterised in that**
it furthermore comprises an accessory stimulating component.

22. A pharmaceutical composition according to claim 21,
**characterised in that**
the accessory stimulating component is selected from cytokines and/or surface antigen B7.

23. Use of a pharmaceutical composition according to any one of claims 20 to 22 for producing an agent for the diagnosis of diseases or a predisposition to diseases which affect the immune system or of tumour diseases or a predisposition to tumour diseases.

24. Use according to claim 23 for producing an agent for the diagnosis of autoimmune diseases or a predisposition to autoimmune diseases.

25. Use according to claim 23 or claim 24 for producing an agent for the diagnosis of diabetes or a predisposition to diabetes.

26. A method for determining a specific T cell subpopulation,
**characterised in that**
a sample containing T cells is brought into contact with a complex according to any one of claims 1 to 19 and the reaction of T cells in the sample with the complex is determined.

27. A method according to claim 26,
**characterised in that**
the reaction of the T cells with a fluorescently labelled complex is determined by FACS analysis.

28. A method according to claim 26 or claim 27,
**characterised in that**
selection for preactivated T cells is carried out before and/or after contact of the T cells with the complex.

29. Use of a pharmaceutical composition according to any one of claims 20 to 22 for producing an agent for the therapy or prevention of diseases which affect the immune system.

30. Use according to claim 29 for producing an agent for the therapy or prevention of autoimmune diseases.

31. Use according to claim 29 or claim 30 for producing an agent for the therapy or prevention of diabetes.

32. Use of a complex according to any one of claims 1 to 19 for producing an antigen, in particular an immunogen or tolerogen.

33. A method for isolating a specific T cell subpopulation,
**characterised in that**
a sample containing T cells is brought into contact with a complex according to any one of claims 1 to 19, the T cells which react with the peptide are identified and optionally isolated from other T cells.

34. A method according to claim 33,
**characterised in that**
selection for preactivated T cells is carried out before and/or after contact of the T cells with the complex.

35. Use of T cells isolated by the method according to claim 33 or substructures thereof for producing an antigen.

36. Use of T cells isolated by the method according to claim 33 or substructures thereof for producing a medicine for reinjection into the patient from whom the T cells or substructures thereof originally originate.

37. Use according to claim 36,
**characterised in that**
the T cells are inactivated T cells.

38. Use according to claim 36,
**characterised in that**
the T cells are T cells which are capable of division.

39. An antibody against a complex according to any one of claims 1 to 19 obtainable by immunisation with the complex and recovery of an antibody produced by the immunisation.

40. An anti-idiotypic antibody against an antibody according to claim 39 obtainable by immunisation with the antibody against the complex and recovery of an anti-idiotypic antibody produced by the immunisation.

41. A T cell which reacts with a complex according to any one of claims 1 to 19.

42. A T cell according to claim 41 which originates from T cell line 6/7 (DSM ACC2172) or has an equivalent T cell receptor binding specificity.

43. A T cell according to claim 41 which originates from T cell line 6/10 (DSM ACC2173) or has an equivalent T cell receptor binding specificity.

## Revendications

1. Complexe, qui comprend au moins un peptide ou un dérivé de peptide lié à une molécule du complexe majeur d'histocompatibilité (CMH) ou à un dérivé d'une molécule du CMH capable de lier un peptide, dans lequel le peptide ou le dérivé de peptide présente une longueur maximale de 100 acides aminés et comprend :
(a) une des séquences d'acides aminés indiquées dans SEQ ID NO 1 à SEQ ID NO 23,
(b) des zones partielles des séquences d'acides aminés indiquées dans SEQ ID NO 1 à SEQ ID NO 23, d'une longueur d'au moins 6 acides aminés, qui présentent une spécificité ou/et une affinité de liaison aux molécules du CMH humain essentiellement équivalentes à celles des séquences d'acides aminés de (a), ou/et
(c) des séquences d'acides aminés dérivées des séquences d'acides aminés indiquées dans SEQ ID NO 1 à SEQ ID NO 23, qui présentent une spécificité
ou/et une affinité de liaison aux molécules du CMH humain essentiellement équivalentes à celles des séquences d'acides aminés de (a), les séquences d'acides aminés dérivées des séquences d'acides aminés indiquées dans SEQ ID NO 1 à SEQ ID NO 23 présentant une homologie de séquence d'au moins 30 % aux séquences d'acides aminés de départ.

2. Complexe selon la revendication 1, dans lequel le peptide ou le dérivé de peptide comprend:
(a) la séquence d'acides aminés indiquée dans la SEQ ID NO 1,
(b) la séquence d'acides aminés indiquée dans la SEQ ID NO 2,
(c) des domaines partiels des séquences d'acides aminés indiquées dans la SEQ ID NO 1 ou/et dans la SEQ ID NO 2, qui présentent une spécificité ou/et une affinité de liaison aux molécules du CMH humain essentiellement équivalentes à celles des séquences d'acides aminés de (a) ou/et de (b), ou/et
(d) des séquences d'acides aminés dérivées des séquences d'acides aminés indiquées dans la SEQ ID NO 1 ou/et la SEQ ID NO 2, qui présentent une spécificité ou/et une affinité de liaison aux molécules du CMH humain essentiellement équivalentes à celles des séquences d'acides aminés de (a) ou/et de (b), les séquences d'acides aminés dérivées des séquences d'acides aminés indiquées dans la SEQ ID NO 1 ou/et la SEQ ID NO 2 présentant une homologie de séquence d'au moins 30 % aux séquences d'acides aminés de départ.

3. Complexe selon la revendication 1 ou 2, **caractérisé en ce que** le peptide ou le dérivé de peptide présente une longueur d'au moins 8 acides aminés.

4. Complexe selon l'une quelconque des revendications 1 à 3, **caractérisé en ce que** le peptide ou le dérivé de peptide présente une longueur d'au moins 10 acides aminés.

5. Complexe selon l'une quelconque des revendications 1 à 3, **caractérisé en ce que** le peptide ou le dérivé de peptide présente une longueur allant jusqu'à 25 acides aminés.

6. Complexe selon l'une quelconque des revendications 1 à 5, **caractérisé en ce que** le peptide ou le dérivé de peptide porte un groupe de marquage.

7. Complexe qui comporte au moins un peptide mimétique d'un peptide ou d'un dérivé de peptide comme défini dans la revendication 1 ou 2, qui est lié à une molécule du CMH ou à un dérivé d'une molécule du CMH capable de lier des peptides, **caractérisé en ce que** le peptide mimétique présente une spécificité ou/et une affinité de liaison aux molécules du CMH humain essentiellement équivalentes à celles d'un peptide ou d'un dérivé de peptide selon une des revendications 1 à 6.

8. Complexe selon l'une quelconque des revendications 1 à 7, **caractérisé en ce qu'**il comprend une molécule du CMH de classe II ou un dérivé de celle-ci liant les peptides.

9. Complexe selon la revendication 8, **caractérisé en ce que** la molécule du CMH de classe II est du type DR1, DR2, DR3 ou DR4.

10. Complexe selon la revendication 9, **caractérisé en ce que** la molécule du CMH de classe II est du sous-type DR B 1 0101, DR B 1 0301, DR B 1 0401, DR B 1 0402, DR B1 0404 ou DR B1 1601.

11. Complexe selon la revendication 10, **caractérisé en ce que** la molécule du CMH de classe II est du sous-type DR B 1 0101 1 ou DR B 1 0401.

12. Complexe selon l'une quelconque des revendications 1 à 11, **caractérisé en ce qu'**il comprend une molécule du CMH recombinante ou un dérivé de celle-ci liant les peptides.

13. Complexe selon la revendication 12, **caractérisé en ce qu'**il comprend un dérivé soluble, liant les peptides, d'une molécule du CMH.

14. Complexe selon l'une quelconque des revendications 1 à 13, **caractérisé en ce qu'**il porte un groupe de marquage.

15. Complexe peptide-molécule du CMH oligomérisé selon l'une quelconque des revendications 1 à 14, qui contient au moins 2 molécules du CMH ou dérivés de molécules du CMH, qui sont associés par des interactions covalentes ou non covalentes.

16. Complexe oligomérisé selon la revendication 15, **caractérisé en ce qu'**il contient des complexes peptide-molécule du CMH réticulés par des réactifs chimiques de couplage.

17. Complexe oligomérisé selon la revendication 15, **caractérisé en ce qu'**il contient des molécules du CMH ou des dérivés de molécules du CMH réticulés par un composant peptidique oligomérisé avec plusieurs zones de liaison au CMH.

18. Complexe oligomérisé selon la revendication 15, **caractérisé en ce qu'**il contient des complexes peptide-molécule du CMH réticulés par des anticorps.

19. Complexe oligomérisé selon l'une quelconque des revendications 15 à 18, **caractérisé en ce qu'**il contient des molécules du CMH telles que définies dans l'une quelconque des revendications 8 à 13.

20. Composition pharmaceutique, **caractérisée en ce qu'**elle contient, comme composant actif, un complexe selon l'une quelconque des revendications 1 à 19, éventuellement en combinaison avec des adjuvants pharmaceutiques habituels.

21. Composition pharmaceutique selon la revendication 20, **caractérisée en ce qu'**elle comprend en outre un composant stimulant accessoire.

22. Composition pharmaceutique selon la revendication 21, **caractérisée en ce que** le composant stimulant accessoire est choisi parmi les cytokines ou/et l'antigène de surface B7.

23. Utilisation d'une composition pharmaceutique selon l'une quelconque des revendications 20 à 22 pour la préparation d'un agent de diagnostic d'affections ou d'une prédisposition à des affections qui influencent le système immunitaire, ou d'affections tumorales ou d'une prédisposition à des affections tumorales.

24. Utilisation selon la revendication 23 pour la préparation d'un agent pour le diagnostic d'affections auto-immunes ou d'une prédisposition à des affections auto-immunes.

25. Utilisation selon la revendication 23 ou 24 pour la préparation d'un agent pour le diagnostic du diabète ou d'une prédisposition au diabète.

26. Procédé pour la détermination d'une sous-population spécifique de cellules T, **caractérisé en ce que** l'on met un échantillon contenant des cellules T en contact avec un complexe selon l'une quelconque des revendications 1 à 19 et **en ce que** l'on détecte avec le complexe la réaction des cellules T dans l'échantillon.

27. Procédé selon la revendication 26, **caractérisé en ce que** l'on détecte la réaction des cellules T par analyse FACS avec un complexe marqué par fluorescence.

28. Procédé selon la revendication 26 ou 27, **caractérisé en ce que** l'on effectue avant et/ou après le contact des cellules T avec le complexe une sélection de cellules T préactivées.

29. Utilisation d'une composition pharmaceutique selon l'une quelconque des revendications 20 à 22 pour la préparation d'un agent pour la thérapie ou la prévention d'affections qui influencent le système immunitaire.

30. Utilisation selon la revendication 29 pour la préparation d'un agent pour la thérapie ou la prévention d'affections auto-immunes.

31. Utilisation selon la revendication 23 ou 24 pour la préparation d'un agent pour la thérapie ou la prévention du diabète.

32. Utilisation d'un complexe selon l'une quelconque des revendications 1 à 19 pour la préparation d'un antigène, en particulier d'un immunogène ou d'un tolérogène.

33. Procédé d'isolement d'une sous-population spécifique de cellules T, **caractérisé en ce que** l'on amène un échantillon contenant des cellules T en contact avec un complexe selon l'une quelconque des revendications 1 à 19, que l'on identifie les cellules T réagissant avec le peptide ou le complexe et, le cas échéant, que l'on les sépare des autres cellules T.

34. Procédé selon la revendication 33, **caractérisé en ce que** l'on effectue une sélection de cellules préactivées avant ou/et après la mise en contact des cellules T avec le complexe.

35. Utilisation des cellules T isolées selon la revendication 33 ou de structures partielles de ces dernières pour la préparation d'un antigène.

36. Utilisation des cellules T isolées selon la revendication 33 ou de structures partielles de ces dernières pour la préparation d'un médicament destiné à être réinjecté dans le patient duquel proviennent à l'origine les cellules T ou les structures partielles de celles-ci.

37. Utilisation selon la revendication 36, **caractérisée en ce que** les cellules T sont des cellules T inactivées.

38. Utilisation selon la revendication 36, **caractérisée en ce que** les cellules T sont des cellules T capables de se multiplier.

39. Anticorps dirigé contre un complexe selon l'une quelconque des revendications 1 à 19, que l'on peut obtenir par immunisation avec le complexe et obtention d'un anticorps produit par l'immunisation.

40. Anticorps anti-idiotypique dirigé contre un anticorps selon la revendication 39, que l'on peut obtenir par immunisation avec l'anticorps dirigé contre le complexe et obtention d'un anticorps anti-idiotypique produit par l'immunisation.

41. Cellule T qui réagit avec un complexe selon l'une quelconque des revendications 1 à 19.

42. Cellule T selon la revendication 41, qui provient de la lignée de cellules T 6/7 (DSM ACC2172) ou qui présente une spécificité équivalente de liaison au récepteur de cellule T.

43. Cellule T selon la revendication 41, qui provient de la lignée de cellules T 6/10 (DSM ACC2173) ou qui présente une spécificité équivalente de liaison au récepteur de cellule T.
